(19) Europäisches Patentamt / European Patent Office / Office européen des brevets

(11) **EP 3 908 670 B1**

(12) **EUROPEAN PATENT SPECIFICATION**

(45) Date of publication and mention of the grant of the patent:
**29.07.2026 Bulletin 2026/31**

(21) Application number: **19909194.3**

(22) Date of filing: **12.04.2019**

(51) International Patent Classification (IPC):
**G01V 5/00** (2024.01)    **G01T 1/20** (2006.01)
**A61B 6/00** (2024.01)    **A61B 6/40** (2024.01)
**C12Q 1/68** (2018.01)

(52) Cooperative Patent Classification (CPC):
**H10F 39/806; G01T 1/20; G01T 3/06; G01V 5/22; G01V 5/222; H10F 39/189; H10F 39/195; A61B 6/4021; A61B 6/4208**

(86) International application number:
**PCT/US2019/027242**

(87) International publication number:
**WO 2020/145999 (16.07.2020 Gazette 2020/29)**

(54) **SPECTRAL DISCRIMINATION USING WAVELENGTH-SHIFTING FIBER-COUPLED SCINTILLATION DETECTORS**

SPEKTRALE DISKRIMINIERUNG UNTER VERWENDUNG VON FASERGEKOPPELTEN SZINTILLATIONSDETEKTOREN MIT WELLENLÄNGENVERSCHIEBUNG

DISCRIMINATION SPECTRALE À L'AIDE DE DÉTECTEURS DE SCINTILLATION COUPLÉS À DES FIBRES À DÉCALAGE DE LONGUEUR D'ONDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.01.2019 US 201916242163**

(43) Date of publication of application:
**17.11.2021 Bulletin 2021/46**

(73) Proprietor: **American Science & Engineering, Inc.**
**Billerica, MA 01821 (US)**

(72) Inventors:
• **COUTURE, Aaron, J.**
  **Reading, MA 01867 (US)**
• **DENKER, Jeffrey, M.**
  **Woburn, MA 01801 (US)**

(74) Representative: **Murgitroyd & Company**
**165-169 Scotland Street**
**Glasgow G5 8PL (GB)**

(56) References cited:
| EP-A1- 1 113 291 | US-A- 5 289 510 |
| US-A- 5 600 144 | US-A- 5 812 720 |
| US-A1- 2004 109 653 | US-A1- 2010 270 462 |
| US-A1- 2016 170 044 | US-A1- 2016 170 044 |
| US-A1- 2017 045 630 | US-A1- 2018 038 969 |
| US-B1- 6 407 392 | US-B2- 6 911 251 |
| US-B2- 9 069 084 | US-B2- 9 618 630 |

• HUTCHINSON D P ET AL: "Wavelength-shifting fiber readout of scintillation detectors", 30 June 2001 (2001-06-30), Berlin, pages 1 - 14, XP93025861, Retrieved from the Internet <URL:https://technicalreports.ornl.gov/cppr/y2001/pres/111170.pdf> [retrieved on 20230221]
• MCKNIGHT ET AL: "The flexible embedded-fiber neutron detector", NUCLEAR INSTRUMENTS & METHODS IN PHYSICS RESEARCH. SECTION A, ELSEVIER BV * NORTH-HOLLAND, NL, vol. 586, no. 2, 3 December 2007 (2007-12-03), pages 246 - 250, XP022457692, ISSN: 0168-9002, DOI: 10.1016/J.NIMA.2007.11.044

EP 3 908 670 B1

- ROSE, KATHRYN: "NuMI Off-Axis Experiment",
  2003, pages 1 - 16, XP009529355, Retrieved from
  the Internet <URL:https://slideplayer.com/slide/
  8765673>

**Description**

FIELD

The present specification relates to fiber-coupled scintillation detectors and to methods of their manufacture, and to systems and methods of X-ray inspection employing fiber-coupled scintillation detectors for efficient detection of X-rays.

BACKGROUND

Fiber-coupled scintillation detectors of radiation and particles have been employed over the course of the past 30 years. In some cases, the scintillator is pixelated, consisting of discrete scintillator elements, and in other cases, other stratagems are employed (such as orthogonally crossed coupling fibers) in order to provide spatial resolution. Examples of fiber-coupled scintillation detectors are provided by US Patent Nos. 6,078,052 (to DiFilippo) and 7,326,9933 (to Katagiri et al.). Detectors described both by DiFilippo and Katagiri et al. employ wavelength-shifting fibers (WSF) such that light reemitted by the core material of the fiber may be conducted, with low attenuation, to photo-detectors disposed at a convenient location, often distant from the scintillator itself. Spatial resolution is of particular value in applications such as neutron imaging. Spatial resolution is also paramount in the Fermi Large Area Space Telescope (formerly, GLAST) where a high-efficiency segmented scintillation detector employs WSF readout for detection of high-energy cosmic rays, as described in Moiseev, et al, High efficiency plastic scintillator detector with wavelength-shifting fiber readout for the GLAST Large Area Telescope, Nucl. Instr Meth Phys. Res. A. vol. 583, pp. 372- 81 (2007).

Because of the contexts in which fiber-coupled scintillator detectors have been employed to date, all known fiber-coupled scintillator detectors have counted pulses produced by individual interactions of particles (photons or massive particles) with the scintillator, thereby allowing the energy deposited by the incident particle to be ascertained based on the cumulative flux of light reemitted by the scintillator.

The detection requirements of X-ray backscatter inspection systems, however, are entirely different from the requirements addressed by existing fiber-coupled scintillation detectors. Backscatter X-ray inspection systems have been used for more than 25 years to detect organic materials concealed inside baggage, cargo containers, in vehicles, and on personnel. Because organic materials in bulk preferentially scatter X rays (by Compton scattering) rather than absorb them, these materials appear as brighter objects in backscatter images. Insofar as incident X-rays are scattered into all directions, sensitivity far overrides spatial resolution as a requirement, and in most scatter applications, detector spatial resolution is of no concern at all, since resolution is governed by the incident beam rather than by detection.

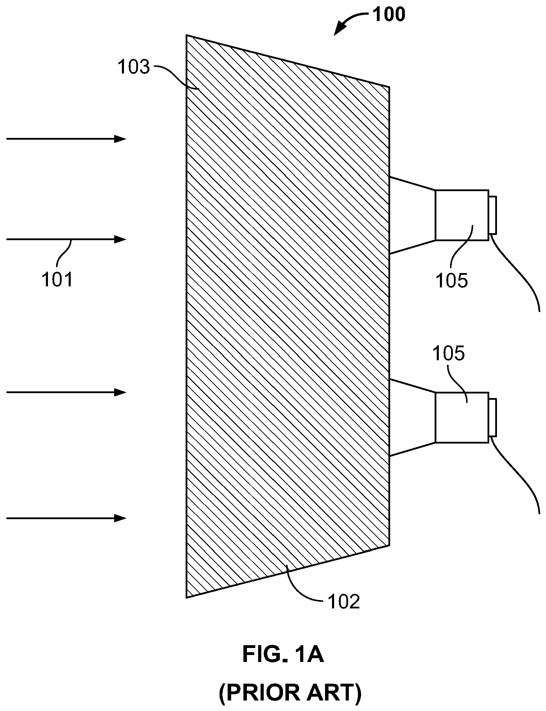

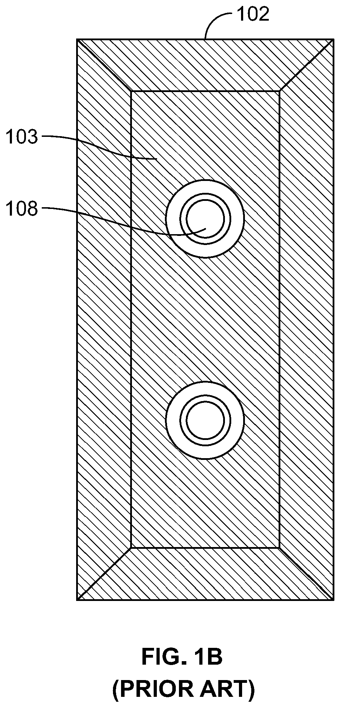

The specialized detection requirements of large area and high sensitivity posed by X-ray scatter systems are particularly vexing in the case of "conventional" scintillation detectors 100 of the type shown in a side cross-section in FIG. 1A and in a front cross-section in FIG. 1B. An example of such a detector is described in US Patent No. 5,302,817 (to Yokota). Typically, a light-tight box 102 is lined with scintillating screens 103 where incident X-ray radiation 101 is converted to scintillation light, typically in the UV, visible, or longer wavelength, portions of the electromagnetic (EM) spectrum. Large-photocathode-area photomultiplier tubes (PMTs) 105 are coupled to receive scintillation light via portholes 108. One problem lies in that a fraction of the scintillation light originating within the screen is transmitted from the screen into the enclosed volume. The remaining scintillation light is lost in the screen material. Scintillating screens 103 are designed to maximize the fraction of emitted light, which is tantamount to ensuring a large transmission coefficient T for the interface between screen 103 and the medium (typically air) filling the detector volume. However, in a conventional backscatter detector of the sort depicted in FIGS. 1A and 1B, the scintillation screens 103 should also serve as good reflectors because scintillation light, once emitted into the volume of box 102, typically needs multiple reflections until it reaches a photo-detector 105. So, the reflection coefficient R of the screen surface should also be large; however, since the sum of T and R is constrained to be unity, both T and R cannot be maximized simultaneously, and a compromise must be struck. As a result, the light collection efficiency of the conventional backscatter detector is inherently low, with only a few percent of the generated scintillation light collected into the photo detectors.

For an imaging detector, the photon statistical noise is calculated in terms of the photons absorbed by the detector and used to generate the image. Any photons which pass through the detector without being absorbed, or even those that are absorbed without generating image information, are wasted and do not contribute to reducing noise in the image. Since photons cannot be subdivided, they represent the fundamental quantum level of a system. It is common practice to calculate the statistical noise in terms of the smallest number of quanta used to represent the image anywhere along the imaging chain. The point along the imaging chain where the fewest number of quanta are used to represent the image is called a "quantum sink". The noise level at the quantum sink determines the noise limit of the imaging system. Without increasing the number of information carriers (i.e., quanta) at the quantum sink, the system noise limit cannot be improved. Poor light collection can possibly create a secondary quantum sink, which is to say that it will limit the fraction of incident X rays resulting in PMT current. Moreover, it will increase image noise.

Light collection efficiency can be improved by increasing the sensitive area of the photo-detectors, however, that path to efficiency is costly.

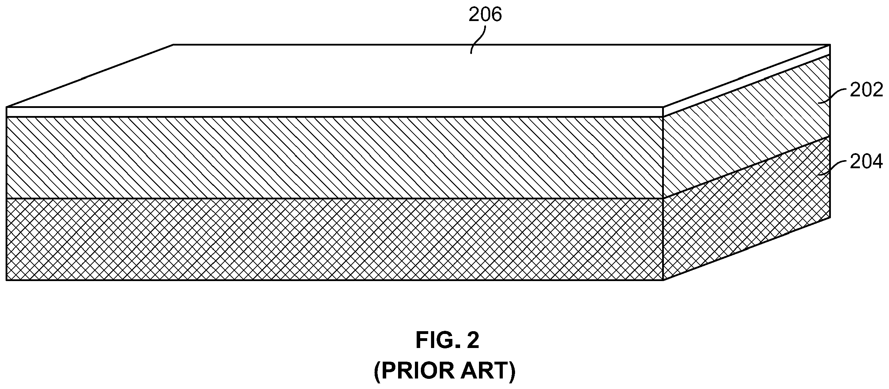

The structure of a scintillating screen typically employed in prior art X-ray scintillation detectors is now described with reference to FIG. 2. A layer of composite scintillator 202 is sandwiched between a backer sheet 204 for structural support and a thin, transparent protective film 206 composed of polyester, for example. The composite scintillator consists of typically micron-size inorganic crystals in an organic matrix or resin. The crystals are the actual scintillating material. Barium fluoro-chloride (BaFCl, or "BFC") or gadolinium oxysulfide ($Gd_2O_2S$, or "Gadox") doped with rare earth elements are common choices for these. The stopping power of the screen is determined by the thickness of the composite scintillator layer 202, which is typically measured in milligrams of scintillator crystal per unit area. Because the inorganic scintillators (such as BFC or Gadox) suffer from high self-ab sorption, the composite scintillator layer has to be kept rather thin in order to extract a good fraction of the scintillation light. This limits the useful stopping power of the screen and makes it suitable only for the detection of X rays with energies up to around 100 keV. It would be advantageous to have scintillation detectors for X-ray scatter detection applications that provide for more efficient extraction, collection, and detection of scintillation light.

Scintillator structures have been produced using many manufacturing technologies, including, for example, die-casting, injection molding (as described by Yoshimura et al ., Plastic scintillator produced by the injection-molding technique, Nucl. Instr Meth Phys. Res. A. vol. 406, pp. 435-41 (1998), and extrusion, (as described in US Patent No. 7,067,079, to Bross, et al).

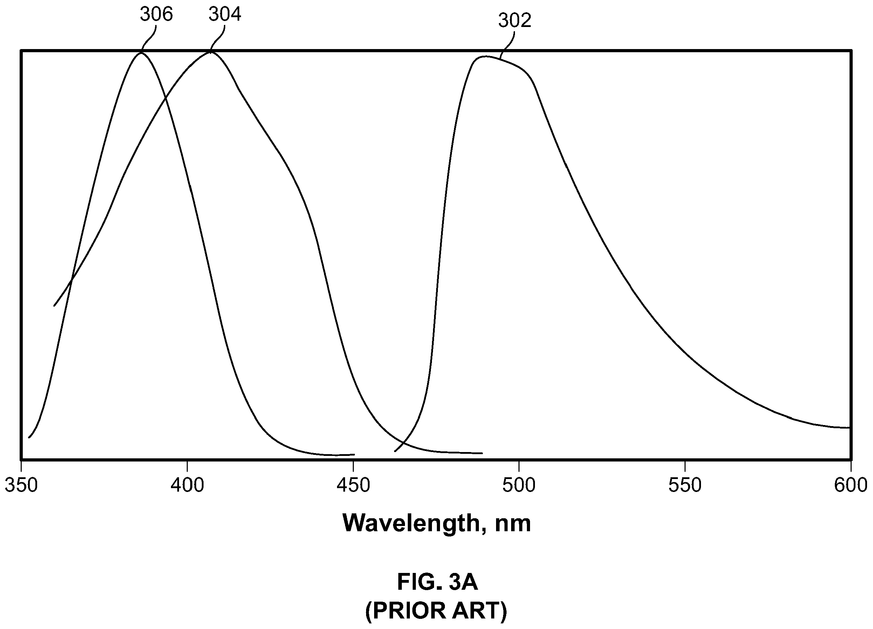

As briefly discussed above, wavelength-shifting fibers (WSF) have long been employed for scintillation detection. Wavelength shifting fibers consist of a core with relatively high refractive index, surrounded by one or more cladding layers of lower refractive index. The core contains wavelength-shifting material, also referred to as dye. Scintillation light which enters the fiber is absorbed by the dye which, in turn, emits light with a longer wavelength. The longer wavelength light is emitted isotropically in the fiber material. Total internal reflection traps a fraction of that light and conducts it over long distances with relatively low loss. This is possible, as described with reference to FIG. 3A, because the absorption 304 and emission 302 wavelength ranges of the dye effectively do not overlap so that the wavelength-shifted light is not reabsorbed.

The captured fraction is determined by the ratio of the refractive indices at surfaces of the fiber. An additional advantage of WSF is that the wavelength shifting can bring the scintillation light 306 into the sensitive wavelength range of the photo detector (PMT, silicon photomultiplier (SiPM), or Multiple-Pixel Photon-Counter (MPPC), or otherwise).

The use of WSF detectors in a flying spot X ray imaging system is known. A flying-spot scanner (FSS) uses a scanning source that is a spot of light, such as but not limited to, a high-resolution, high-light-output, low-persistence cathode ray tube (CRT), to scan an image. In contrast with film or digital X-ray detectors which have spatially sensitive detectors that establish the system resolution, flying spot X-ray systems are limited by the illumination beam spot size. The illumination beam spot size is determined by a number of factors including the X-ray focal spot size, the collimation length, the aperture size and the distance to the target.

The beam spot is the pinhole image of the focal spot, geometrically blurred by the relatively large size of the pinhole or aperture. In general, the shape of the aperture is substantially similar to the shape of the focal spot but typically larger. Accordingly, any internal structure is blurred out and only the overall dimension of the focal spot is relevant. The ideal beam spot is a sharp disk or rectangle. In reality, however, the edges are blurred. The umbra region is obtained by the projection of the aperture from the equivalent/virtual point source, which is, however, only well-defined for the case of round disk-shaped focal spot. The umbra region is defined as the region in which the entirety of the light source is obscured by the occluding body being imaged; while the penumbra is the image region where in which only a portion of the light source is obscured by the occluding body.

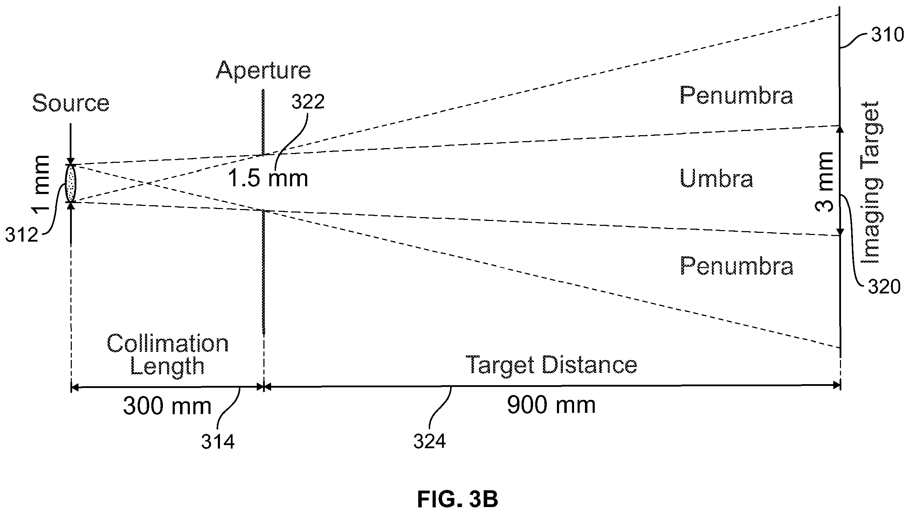

The actual two-dimensional intensity distribution describing the beam spot is controlled by the combination of both the focal spot and the collimator. Known collimation designs strive to minimize the size of the penumbra, as shown in FIG. 3B, which illustrates the relationship between focal length and umbra and penumbra diameters with the light and dark regions reversed. As shown, a width 310 of the penumbra correlates to and scales with the ratio of a focal spot 312 size to collimation length 314, which is the distance between focal spot 312 and aperture 316, as defined by the source 318 and the target 320. Consequently, a small focal spot allows for a shorter collimator (more compact and lighter design) and/or better collimation (smaller penumbra).

Mathematically, an umbra diameter (UD) 320 and a penumbra width (PW) 310 are related to the diameter of focal spot 312 (FS), the diameter of the aperture (AD) 322, collimation length (CL) 314, and target distance (TD) 324 through the following equations:

$$UD = AD + \frac{TD}{CL}(AD - FS) \qquad (1)$$

$$PW = FS * \frac{TD}{CL} \qquad (2)$$

The resolution of currently available flying spot X-ray imaging systems is limited by the size of the flying spot. The detection system has little to no spatial sensitivity, and as a result, the spatial information is created by moving the spot across the detector with synchronization to the detector readout over time. The minimum spot size is limited by the X-ray source spot size and the collimation system used to generate the spot. Typically, in cargo imaging systems, the spot is 7- 10 mm in size at the detector. As shown by the equations (1) and (2) above, reducing the size of the focal spot enables designing short length collimators for flying spot X-ray imaging systems and obtaining sharper images.

US 2016/170044 discloses methods for discriminating among x-ray beams of distinct energy content. A first volume of scintillation medium converts energy of incident penetrating radiation into scintillation light which is extracted from a scintillation light extraction region by a plurality of optical waveguides that convert the scintillation light to light of a longer wavelength. An x-ray beam initially incident upon the first volume of scintillation medium and traversing the first volume is then incident on a second volume of scintillation medium. Scintillation light from the first and second scintillation volumes is detected in respective detectors and processed to yield a measure of respective low energy and high-energy components of the incident x-ray beam.

US6,407,392 discloses a radiation detector comprising a scintillator, a first light guide, a plurality of second light guides and a photo detector. The scintillator generates a scintillated light in response to received radiation. The first light guide, which is connected to the scintillator, has a fluorescence characteristic. The second light guide has a common surface arranged at the opposite side of the surface of the scintillator where radiation is received, and the second light guide has a fluorescence characteristic. The photo detector is connected to the first light guide and the second light guide and detects a fluorescent light therein.

US9,618, 630 discloses a radiation detection system comprising a detection grid of wavelength shifting fibers with a volume of scintillating material at the intersecting points of the fibers. Light detectors, preferably Silicon Photomultipliers, are positioned at the ends of the fibers. The position of radiation is determined from data obtained from the detection grid.

US5,289,510 discloses nuclear reaction detectors capable of position sensitivity with submillimeter resolution in two dimensions by placing arrays of scintillation or wavelength shifting optical fibers formed of a plurality of such optical fibers in a side-by-side relationship in X and Y directions with a layer of nuclear reactive material operatively associated with surface regions of the optical fiber arrays. Each nuclear reaction occurring in the layer of nuclear reactive material produces energetic particles for simultaneously providing a light pulse in a single optical fiber in the X oriented array and in a single optical fiber in the Y oriented array. These pulses of light are transmitted to a signal producing circuit for providing signals indicative of the X-Y coordinates of each nuclear event.

US9,069,084 discloses a radiation detection apparatus including a radiation sensor having a corresponding radiation sensing region, and a photosensor that is optically coupled to the radiation sensor. The radiation sensing region includes optical fibers. Some or all of the optical fibers are coated. The coating includes a phosphorescent material. The optical fibers can be arranged in a manner such that optical substrates have substantially no bends.

In light of the above, there is clearly a need for increased spatial sensitivity for X-ray detectors in a flying spot imaging system. There is also a need to develop a WSF system that is capable of determining both the high resolution content of an image as well as the low resolution mapping of the coarse location of the spot. Furthermore, there is a need to be able to generate a high resolution image with a minimum of individual channels, thus saving cost and complexity of the system. Finally, there is a need for an improved detection system that could be effectively used in any flying spot x-ray system and configured to generate improved resolution in one or two dimensions.

For convenience of notation, a wavelength-shifted fiber-coupled scintillation detector may be referred to herein as an "Sc-WSF" detector.

According to the present invention, there is provided a detector for an X-ray imaging system according to the appended claims 1-12.

## BRIEF DESCRIPTION OF THE DRAWINGS

These and other features and advantages of the present specification will be further appreciated, as they become better understood by reference to the detailed description when considered in connection with the accompanying drawings:

FIGS. 1 A and 1B show side and front cross-sectional views, respectively, of a "box - type" prior art scintillation detector;

FIG. 2 is a schematic view of a prior art scintillator screen;

FIG. 3A depicts spectral relationships among scintillation light and typical wavelength-shifting fiber absorption and emission spectra;

FIG. 3B illustrates the relationship between focal length and umbra and penumbra diameters with light and dark regions reversed;

FIG. 4 is a perspective schematic view of an array of wavelength-shifting fibers sandwiched between scintillator material, in accordance with an embodiment of the present specification;

FIG. 5 is a cross-sectional schematic view of an array of wavelength-shifting fibers embedded within a matrix of scintillator material, as an example not encompassed by the appended claims but useful for understanding the invention;

FIG. 6 A is a perspective view of a cylindrical scintillator extruded about a WSF, in accordance with an embodiment of the present specification;

FIG. 6B is a schematic depiction of a system for extruding a cylindrical scintillator about a WSF, in accordance with an embodiment of the present specification;

FIG. 6C is a cross-sectional view of an extruder for co-extruding a cylindrical scintillator with a WSF, in accordance with an embodiment of the present specification;

FIG. 7A is a schematic cross-section of a scintillation detector with multiple rows of WSF, in accordance with an embodiment of the present specification;

FIG. 7B illustrates a schematic cross-section of a scintillation detector with multiple rows of WSF, in accordance with another embodiment of the present specification;

FIG. 8 is a top view of a wavelength-shifted fiber-coupled scintillation detector in accordance with an embodiment of the present specification;

FIG. 9 shows roof and skirt backscatter detectors, stowed in accordance with embodiments of the present specification;

FIG. 10 illustrates the detectors shown in FIG. 9 deployed during the course of inspection operations;

FIG. 11 shows an awning detector and a skirt detector for use with a backscatter inspection system in accordance with embodiments of the present specification;

FIG. 12 is a cross-sectional schematic view of a stack of scintillator layers for use as a high-energy X-ray transmission detector, in accordance with an embodiment of the present specification;

FIG. 13A shows a layered transmission detector inside a 2-inch-high speed bump, in accordance with an embodiment of the present specification;

FIG. 13B shows a layered transmission detector inside a 2-inch-high speed bump, in accordance with an embodiment of the present specification;

FIG. 13C shows a cross-section of the detector assembly shown in FIGS. 13A and 13B inserted into the speed bump frame;

FIG. 14A shows a perspective view of a segmented X-ray transmission detector for measurement of the distribution of detected intensity across the width of an X-ray beam, in accordance with an embodiment of the present specification;

FIG. 14B shows an end-on cross-section and a typical beam profile of the detector of FIG. 14A;

FIG. 14C shows an end-on cross-section and a typical beam profile of the detector of FIG. 14A;

FIG. 15 is a cross-sectional view of a scintillation detector with multi-energy resolution, in accordance with an embodiment of the present specification;

FIG. 16 shows a multi-layer scintillation detector for detection of both X-rays and thermal neutrons, in accordance with an embodiment of the present specification;

FIG. 17 shows a perspective view of a detector with active collimators;

FIG. 18A shows a perspective view of a WSF-detector used as an active collimator in accordance with an embodiment of the present specification;

FIG. 18B shows a cross-sectional view of a WSF-detector used as an active collimator in accordance with an embodiment of the present specification;

FIG. 18C shows an arrangement with independent readout separated by a light-tight X-ray absorber to distinguish radiation striking each face, in accordance with a further embodiment of the present specification;

FIG. 18D shows an arrangement with independent readout separated by a light-tight X-ray absorber to distinguish radiation striking each face, in accordance with a further embodiment of the present specification;

FIG. 19A shows multiple detectors folding out of a hand-held scanner, in a stored condition, in accordance with an embodiment of the present specification;

FIG. 19B shows multiple detectors folding out of a hand-held scanner, in a deployed condition, in accordance with an embodiment of the present specification;

FIG. 20A shows a backscatter unit that, by virtue of Sc-WSF detectors in accordance with the present specification, may be slid under a vehicle for under-chassis inspection;

FIG. 20B shows a backscatter unit that, by virtue of Sc-WSF detectors in accordance with the present specification, may be slid under a vehicle for under-chassis inspection;

FIG. 21A depicts the use of a right-angled combination of detectors based on Sc-WSF technology in conjunction with a mobile inspection system and in accordance with an embodiment of the present specification;

FIG. 21B depicts the use of a right-angled combination of detectors based on Sc-WSF technology in conjunction with a mobile inspection system and in accordance with an embodiment of the present specification;

FIG. 22A illustrates a WSF detector with enhanced resolution, in accordance with an embodiment of the present specification;

FIG. 22B illustrates a graph depicting the fiber response uniformity of the detector shown in FIG. 22A;

FIG. 22C illustrates a high resolution fiber layout, in accordance with an embodiment of the present specification;

FIG. 23 illustrates X-ray absorption and light collection in a high resolution layer of a WSF detector, in accordance with an embodiment of the present specification;

FIG. 24A illustrates a 16 channel PMT coupling signals obtained from all high resolution fibers and low resolution fibers of the detector illustrated in FIG. 22A, in accordance with an embodiment of the present specification;

FIG. 24B is a diagrammatical representation of a 64 channel multi-anode PMT assembly that may be used for reading light signals captured by the WSF detector of the present specification;

FIG. 25 is a block diagram illustrating the layers of an exemplary multi-layer high-resolution detector, in accordance with an embodiment of the present specification;

FIG. 26A illustrates a detector panel placed in direct beam of scanning radiation emitted by a small portable scanner being used to scan an object, in accordance with an embodiment of the present specification;

FIG. 26B illustrates a backscatter image obtained by the scanner of FIG. 26A, in accordance with an embodiment of the present specification;

FIG. 26C illustrates a transmission image obtained by a built-in detector of the scanner of FIG. 26A by using the detector panel as shown in FIG. 26A, in accordance with an embodiment of the present specification;

FIG. 26D illustrates transmission images of a gun placed behind steel walls of different thickness obtained by using the detector panel shown in FIG. 26A;

FIG. 27A illustrates a diagrammatical representation of a WSF detector panel, in accordance with another embodiment of the present specification;

FIG. 27B illustrates the WSF detector panel of FIG. 27A, in accordance with an embodiment of the present specification; and

FIG. 28 is a flowchart illustrating the steps in a method of forming a detector with at least one high resolution layer and at least one low resolution layer, in accordance with some embodiments of the present specification.

## DETAILED DESCRIPTION

In accordance with embodiments of the present specification, the optical coupling of scintillator material to optical waveguides, and, more particularly, to wavelength-shifting fibers, advantageously enables objectives including those peculiar to the demands of X-ray scatter detection.

The term "image" shall refer to any unidimensional or multidimensional representation, whether in tangible or otherwise perceptible form, or otherwise, whereby a value of some characteristic (such as fractional transmitted intensity through a column of an inspected object traversed by an incident beam, in the case of X-ray transmission imaging) is associated with each of a plurality of locations (or, vectors in a Euclidean space, typically R2) corresponding to dimensional coordinates of an object in physical space, though not necessarily mapped one-to-one thereonto. An image may comprise an array of numbers in a computer memory or holographic medium. Similarly, "imaging" refers to the rendering of a stated physical characteristic in terms of one or more images.

For purposes of the present description, in some embodiments, a 'high resolution layer' is defined as a layer of a detector comprising a first plurality of wavelength-shifting optical fibers, wherein each of the first plurality of wavelength-shifting optical fibers is defined by a first fiber radius and a first spacing between adjacent ones of the first plurality of wavelength-shifting optical fibers, wherein each of the first plurality of wavelength-shifting optical fibers extends through a detection region and under a scintillation screen of the detector, and wherein the first plurality of wavelength-shifting optical fibers is configured to receive radiation and generate signals.

For purposes of the present description, in some embodiments, a 'low resolution layer' is defined as a layer of a detector comprising a second plurality of wavelength-shifting optical fibers wherein each of the second plurality of wavelength-shifting optical fibers is defined by a second fiber radius and a second spacing between adjacent ones of the second plurality of wavelength-shifting optical fibers, and wherein at least one of the second fiber radius is larger than the first fiber radius of the 'high resolution layer' or the second spacing is greater than the first spacing of the 'high resolution layer', and wherein the second plurality of wavelength-shifting optical fibers is configured to receive the radiation that passes through the 'high resolution layer' and generate signals.

For purposes of the present description, and in any appended claims, the term "thickness," as applied to a scintillation detector, shall represent the mean extent of the detector in a dimension along, or parallel to, a centroid of the field of view of the detector. The term area, as applied to a detector, or, equivalently, the term "active area" shall refer to the size of the detector measured in a plane transverse to centroid of all propagation vectors of radiation within the field of view of the detector.

Terms of spatial relation, such as "above," "below," "upper," "lower," and the like, may be used herein for ease of description to describe the relationship of one element to another as shown in the figures. It will be understood that such terms of spatial relation are intended to encompass different orientations of the apparatus in use or operation in addition to the orientation described and/or depicted in the figures.

As used herein, and in any appended claims, the term "large-area detector" shall refer to any single detector, or to any detector module, subtending an opening angle of at least 30° in each of two orthogonal transverse directions as viewed from a point on an object undergoing inspection, equivalently, characterized by a spatial angle of at least $\pi$ steradians.

A "conveyance" shall be any device characterized by a platform borne on ground-contacting members such as wheels, tracks, treads, skids, etc., used for transporting equipment from one location to another.

Where an element is described as being "on," "connected to," or "coupled to" another element, it may be directly on, connected or coupled to the other element, or, alternatively, one or more intervening elements may be present, unless otherwise specified.

The terminology used herein is for the purpose of describing particular embodiments and is not intended to be limiting. The singular forms "a," "an," and "the," are intended to include the plural forms as well.

In the description and claims of the application, each of the words "comprise" "include" and "have", and forms thereof, are not necessarily limited to members in a list with which the words may be associated. It should be noted herein that any feature or component described in association with a specific embodiment may be used and implemented with any other embodiment unless clearly indicated otherwise.

The present specification is directed towards multiple embodiments. The following disclosure is provided in order to enable a person having ordinary skill in the art to practice the specification. Language used in this specification should not be interpreted as a general disavowal of any one specific embodiment or used to limit the claims beyond the meaning of the terms used therein. The general principles defined herein may be applied to other embodiments and applications without departing from the scope of the specification. Also, the terminology and phraseology used is for the purpose of describing exemplary embodiments and should not be considered limiting. Thus, the present specification is to be accorded the widest scope encompassing numerous alternatives, modifications and equivalents consistent with the principles and features disclosed. For purpose of clarity, details relating to technical material that is known in the technical fields related to the specification have not been described in detail so as not to unnecessarily obscure the present specification.

It should be noted herein that any feature or component described in association with a specific embodiment may be used and implemented with any other embodiment unless clearly indicated otherwise.

## WSF Detectors

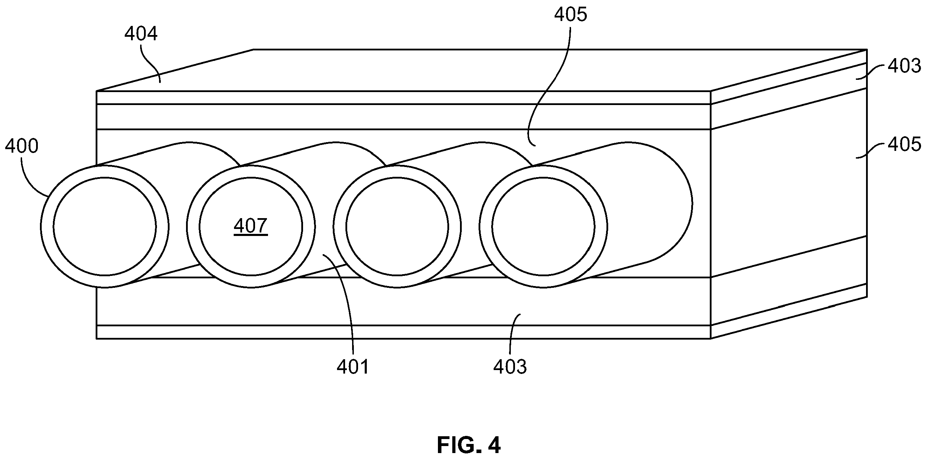

Referring, first, to FIG. 4, in one embodiment of the specification, a layer of closely spaced parallel wavelength-shifting fibers 400 is sandwiched between two layers 403 of composite scintillating screen. The preferred scintillator material is europium-doped barium fluorochloride (BaFCl:Eu), although other scintillators, such as BaFI:Eu, or other lanthanide-doped barium mixed halides (including, by way of further example, BaBrI:Eu and BaCsI:Eu), may be used within the scope of the present specification. Since scintillator materials employed for X-ray detection typically exhibit very strong self-absorption of scintillation photons, embodiments in accordance with the present specification advantageously allow unusually large volumes of scintillator 403 to be employed while still efficiently coupling out scintillation signal.

One advantage to using composite scintillation screen in the present application is that it allows for fabrication by extrusion of a fiber-coupled scintillation detector.

Composite scintillator 403 is structurally supported by exterior layers 404 of plastic, or other material, providing mechanical support. Optical contact between the fiber cladding 401 and the composite scintillator 403 is established by filling the voids with index-matching material 405 of suitable refractive index which is transparent to the scintillation light. The refractive index of the filling material is chosen to optimize the collection of primary light photons into the WSF and the capture of wavelength-shifted photons in the fiber. Filling material 405 may be optical grease or optical epoxy, for example, though any material is within the scope of the present specification.

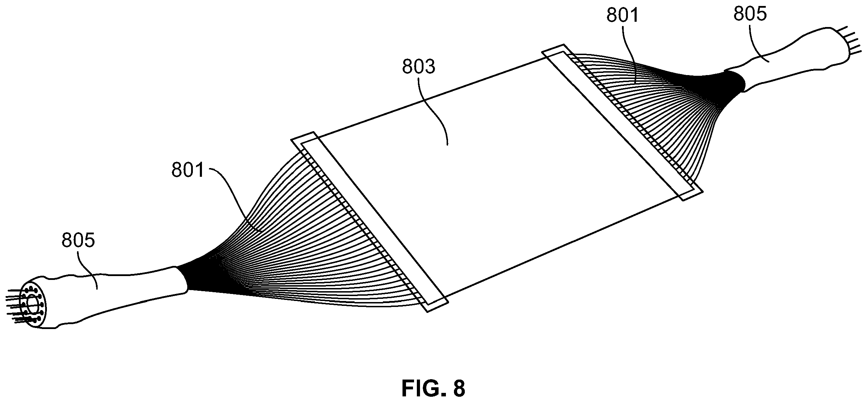

Upon incidence of X-ray photons, scintillation light emitted by scintillator 403 is coupled via cladding 401 into core 407 of the respective fibers, down-shifted in frequency (i.e., red-shifted) and propagated to one or more photo-detectors 805 (shown in FIG. 8, for example). Light from the fiber cores 407 is converted into a current via photo-detector 805, and the current is integrated for an interval of time, typically in the range of 1-12 $\mu$s, to obtain the signal strength for each pixel. Integration of the detector signal may be performed by an integrating circuit (not shown), such as an integrating pre-amplifier, for example.

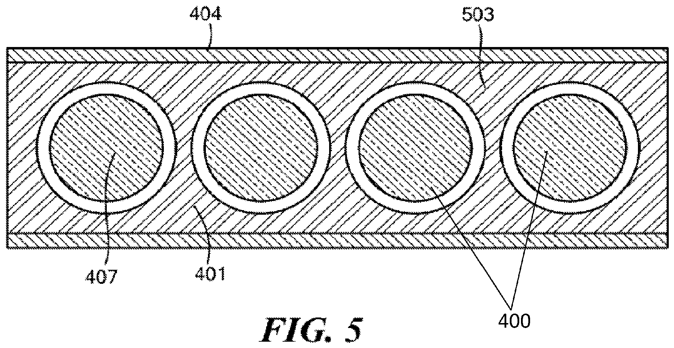

Referring now to FIG. 5, as an example not encompassed by the appended claims, wavelength-shifting fibers 400 are embedded in the matrix of the scintillating screen 503. Embedding the WSF into the scintillating medium creates the best optical contact.

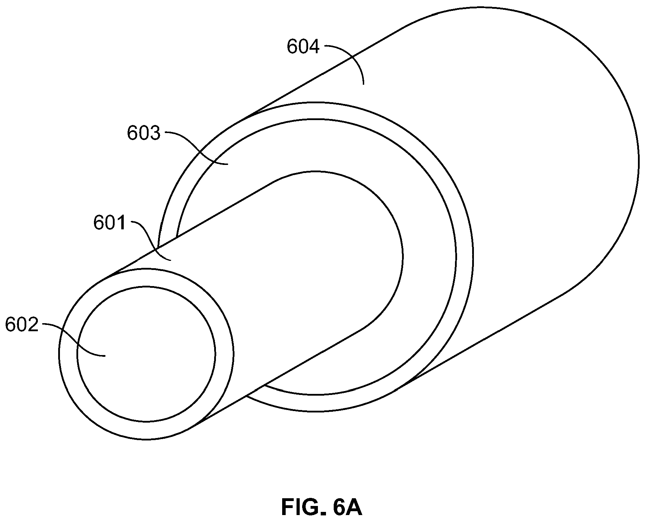

In yet another embodiment of the specification, described now with reference to FIG. 6A, composite scintillator material 603 is applied like a cladding or shell around a WSF 601 with core 602. This application lends itself to an extrusion-style manufacturing process and allows making the most effective use of costly scintillator material 603. The scintillator material 603 is sealed off with a protective layer 604 which also acts as a reflector to the scintillation light. Within the scope of the present specification, the cladding is optional and may be omitted when the scintillator has a lower index of refraction than the fiber and the scintillator-fiber bond has the necessary smoothness and robustness.

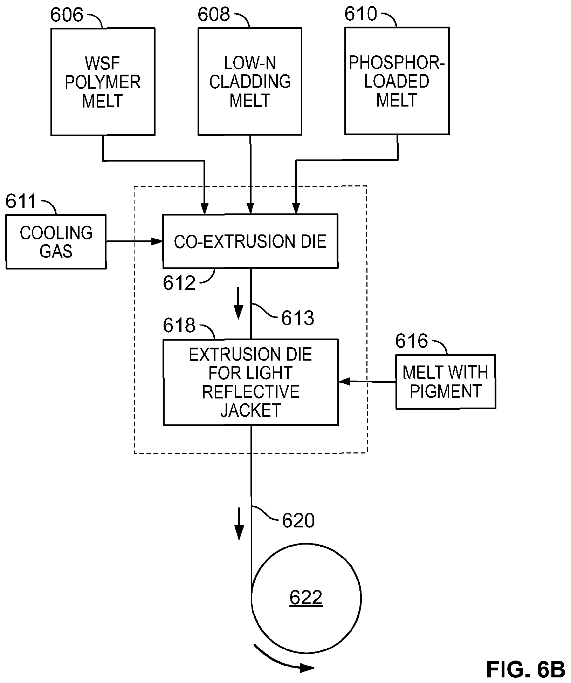

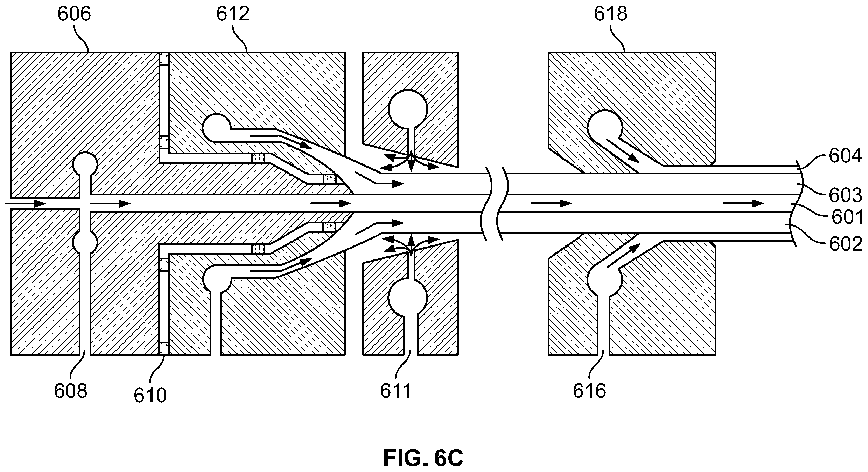

A wavelength-shifting polymer optical fiber may be manufactured, in accordance with an embodiment of the specification now described with reference to the system schematic depicted in FIG. 6B. Sources of WSF polymer melt 606, low-refractive-index cladding polymer melt 608, and phosphor-embedded optically transparent polymer melt 610, all under pressure, are fed into a co-extrusion die 612 within extrusion zone 614, and co-extruded. Dry gas 611, such as dry air or nitrogen, for example, is sprayed onto the extruded fiber for cooling. Polymer melt with a light-reflective pigment (such as $TiO_2$, for example) 616 is fed under pressure into an extrusion die 618 for a light-reflective jacket over the scintillator-coated WSF 613. The resultant scintillator-loaded WSF 620 is wound for storage by winder 622. FIG. 6C shows a cross-sectional view of a co-extrusion system, for use in accordance with embodiments of the present specification, for the manufacture of scintillator-coated WSF. The WSF polymer melt 606 is injected, along with the low-refractive-index cladding polymer melt 608 and phosphor-embedded optically transparent polymer melt 610, into co-extrusion die 612. Polymer melt with a light-reflective pigment 616 is fed under pressure into extrusion die 618. The completed fiber has a WSF core 602, a low-index cladding 601, a scintillator-loaded cladding 603, and a reflective coating 604.

For all embodiments of a scintillation detector in accordance with the present specification, it is advantageous that the thickness of the scintillator material be optimized for the energy of the radiation to be detected. The design should ensure sufficient light collection to avoid a secondary quantum sink. In particular, embodiments of the specification described herein provide for detectors of extraordinary thinness relative to their area.

Embodiments of the present specification, even those with as many as 8 WSF layers, have ratios of the square of detector thickness to the active detector areas that are less than 0.001. For example, an 8-layer detector with an area of 48" × 12" has a thickness no greater than 0.5", such that the ratio of the square of the thickness to the detector area is 0.0005. This thickness-squared-to-area ratio is typically an order of magnitude, or more, smaller than the comparable ratio for backscatter detectors where scintillator light is directly detected by a photo-detector.

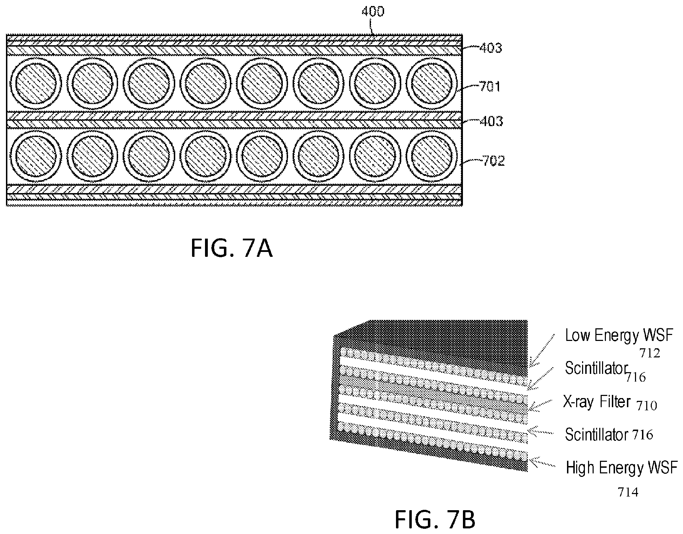

In accordance with a further embodiment of the specification depicted in FIG. 7A, the useful stopping power of the detector can be increased by combining multiple layers 701, 702 of WSF 400 (or - as an example not encompassed by the appended claims-other optical waveguides) thereby increasing the depth of scintillator material 403 along the path of the incident radiation. FIG. 7B illustrates a schematic cross-section of a scintillation detector with multiple rows of WSF, in accordance with another embodiment of the present specification. As shown in the figure, in order to produce image data with materials separation, multiple layers of WSF detectors are stacked, and are separated by a metallic material which functions as a filter and hardens an incident X-ray beam. An X-ray filter 710 is inserted between layers of low energy WSF 712, high energy WSF 714 and scintillator material 716. In an embodiment, copper is used as a filter due to its good performance and cost over alternative materials. In an embodiment, a copper filter having a thickness ranging from 0.5 mm to 1.0 mm is used, which may shift the X-ray beam's peak energy by 20 kV to 30 kV.

An embodiment of a wavelength-shifted scintillator detector in accordance with the present specification is shown in FIG. 8. As an example, not encompassed by the appended claims, wavelength-shifting fibers 801 are embedded within scintillator material 803, coupling light, and downshifting it in frequency for detection by photomultiplier tubes 805. In accordance with various of the embodiments heretofore described, the ends of the WSF are bundled and optically coupled to at least one photo-detector. Examples of suitable photo detectors include PMTs and silicon photomultipliers (SiPMs).

Advantages of the detector, the specification of which is described herein, include the efficiency of detection, and the low geometrical profile of implementation. This allows greater freedom in designing a detection system and it makes entirely new, space constrained applications possible. The mechanical flexibility of the detector structure allows shaping the detector surface to conform to the application, such as an implementation in which an imaged object is surrounded by detector volume. The low profile also makes it relatively easy to orient and shield the detector area in ways to minimize the detection of unwanted scatter radiation (crosstalk) from a nearby X-ray imaging system.

The extraction of scintillation light over a large region of scintillator enables detectors of large width-to-depth aspect ratio. In particular, detectors subtending spatial angles of 0.1 sr, or more, are facilitated by embodiments of the present specification.

In a typical backscatter X-ray imaging system, an X-ray pencil beam scans an imaged target in a linear motion, while elongated radiation detectors are arranged on both sides of an exit aperture of an X-ray source. As the pencil beam moves, the detector area closest to the beam will typically receive the strongest signal and detector area further from the beam less. If the detector area is segmented into individually readable sections the signal to noise ratio of the detection system can be improved by only reading the segments with a good signal to noise ratio and neglecting the segments which would contribute predominantly noise to the summed signal. The selection of contributing detector segments can be made based on the actually detected signal or based on the known position of the pencil beam.

The extrusion, or "automated coating" process, described above with reference to FIGS. 6A-6C, is in stark contrast to typical methods of laying down polycrystalline scintillation material, such as BaFCl(Eu), on a flat backing. The extrusion method of fabricating individual wavelength-shifting fibers coated with a uniform thickness of scintillator, as taught above, produces fibers that can be contoured so that the restrictions on the shape of a Sc-WSF detector is governed primarily by the requirement of full capture in the fiber by total internal reflection. The concept of uniformly coated coupling fibers gives greater freedom to the design of backscatter (BX) detectors, especially hand-held and robot-mounted detectors, where space is at a premium.

Deployable Detectors to Increase the Geometric Efficiency of Scattered X rays:

Some mobile X-ray systems, such as those described, for example, in US Patent Nos. 5,764,683, to Swift, et al. and 7,099,434, to Chalmers et ah, use the method of backscattered X rays (BX) to inspect cars and trucks from one side. The former uses detectors deployed outside a conveyance during operation, whereas the latter uses a detector area entirely contained within an enclosure, namely the skin of a conveyance. Both use large-area detectors to maximize the efficiency of detecting the scattered X rays. The areal backscatter detector coverage in the case of a product in accordance with the teachings of the Chalmers'434 Patent covers on the order of 20 square feet of the interior surface of an enclosure that faces the target. This covert detector area has relatively poor geometrical efficiency for collecting the scattered radiation from high or low targets. The intrinsically deep geometrical profile of such detectors, necessary for direct capture of the scintillation light by photomultipliers, is inimical to deployment outside the van.

An Sc-WSF detector, in accordance with embodiments of the present specification, makes practical the unobtrusive storage of large-area detectors that can be quickly deployed outside the van in positions that substantially enhance detection efficiency.

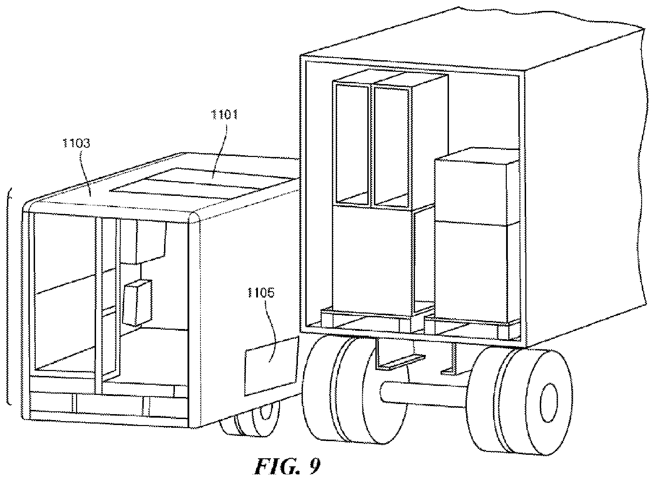

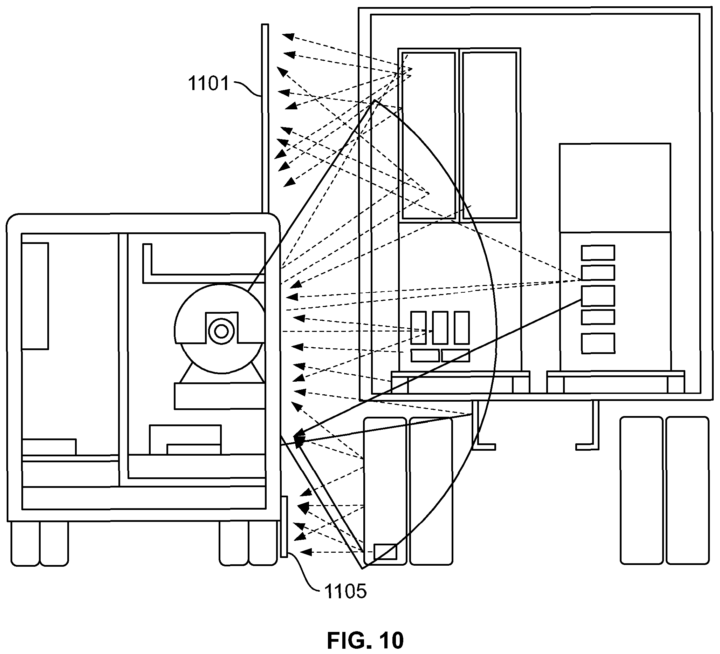

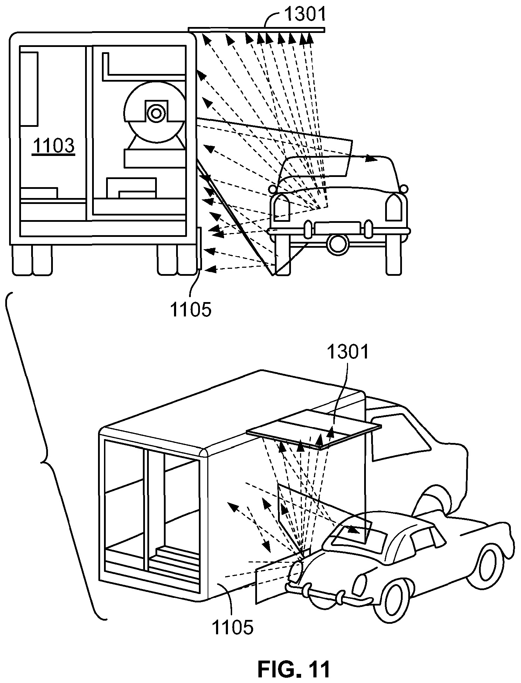

Referring, now, to FIG. 9, a large-area Sc-WSF awning detector 1101 is shown in a stowed position, stored on the roof of a backscatter inspection van 1103, and a thin skirt detector 1105 is shown in a stowed position above a wheel of the backscatter inspection van. In FIG. 10, both the roof and skirt detectors are shown as deployed to increase the solid angle for detecting higher and lower targets, respectively; the awning detector is deployed above an inspected object during the course of inspection, while the skirt detector is deployed, at least in part, beneath the platform of the conveyance. In another embodiment of the specification, described with reference to FIG. 11, an awning detector 1301 may be deployed for low, close targets, such as for detection of contraband in the trunk or far side of a car 1303. Awning detector 1301 may slide out from a roof of the conveyance prior to inspection operation. FIG. 11 also shows the deployment of Sc-WSF skirt detectors 1105 used to efficiently examine the tires, wheel wells, and the interior of close vehicles.

Scanning pencil beams of X rays not only reveal interior objects by analyzing the backscattered radiation but, in some applications, can obtain additional information by the simultaneous analysis of transmission (TX) and forward scattered (FX) radiation. The TX and FX detectors need not be segmented since the cross-sectional area of the pencil beam, together with the integration time of the signal, defines the pixel size. Moreover, the TX and FX detectors only need to be total energy detectors since, in most applications, the flux of the TX or FX X rays is too high for pulse counting. Scintillation screens are the traditional detectors for such scanning beam applications. Sc-WSF detectors substantially extend the range of applications of present TX and FX scintillation detectors, as the following examples make clear.

TX for X-ray beams up to at least 250 keV:

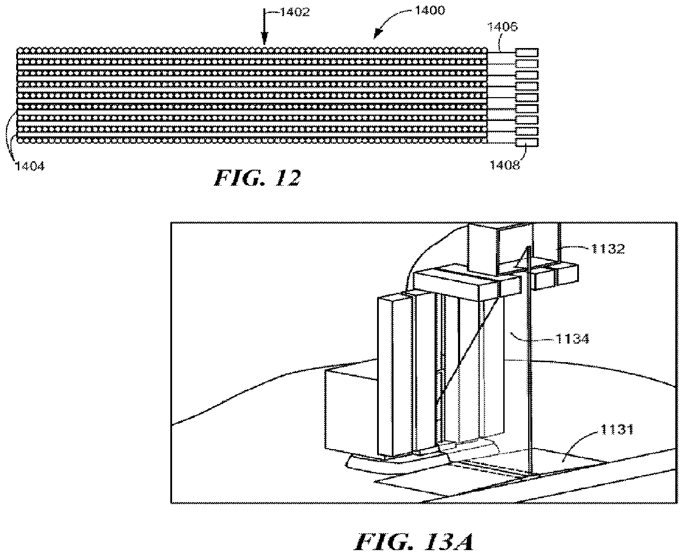

The absorption efficiency of traditional scintillation screens, made, for example, of BaFCl(Eu) or Gadox, drops below 50% for X-ray energies above ~80 keV. The 50% point for two layers is about 100 keV. By way of distinction, Sc-WSF detector can be made with more than two layers of scintillators without substantially increasing the profile of the detector. A cost-effective Sc-WSF detector, with 4 layers, can be used for TX with scanning X-ray beams generated by a standard 140 keV X-ray tube. A multi-layer detector such as the 9-layer detector, as shown in FIG. 12, and designated there generally by numeral 1400, can be highly effective for a detecting X rays 1402 emitted by a standard 225 keV X-ray tube (not shown), such as that used in the X-ray inspection of vehicles through portals. Layers 1404 of scintillator material are shown, and WSF fibers 1406 coupled to photo-detectors 1408.

Transportable TX Detector for a Top-Down Imager in Three-Sided Portal Inspection:

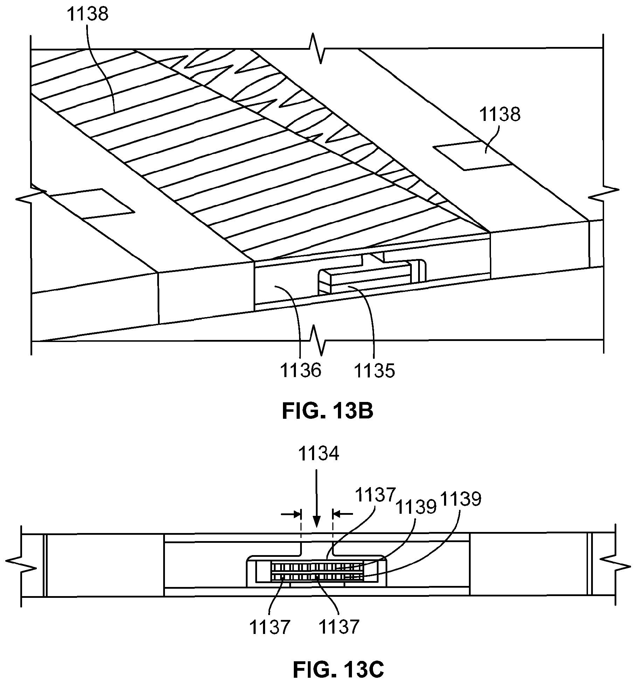

The thin profile of the multi-layer transmission (TX) detector makes practical a top-of-the-road transmission (TX) detector. FIGS. 13A and 13B show such a detector inside a 2-inch-high speed bump 1131 strong enough to support a fully-loaded tractor trailer, and requiring no excavation of the ground for deployment. Source 1132 of penetrating radiation emits fan beam 1134 incident upon a linear detector assembly 1135 within frame 1136 of speed bump 1131 or a similar protrusion above an underlying surface. Detector assembly 1135 includes segments of scintillator material 1137 separated by vanes 1138 of high atomic number. As described above, for example with reference to FIG. 4, scintillation light is coupled to photodetectors by means of wave-length shifting optical fibers 1139.

Segmented TX Detector for Determining the Scan Beam Intensity Profile:

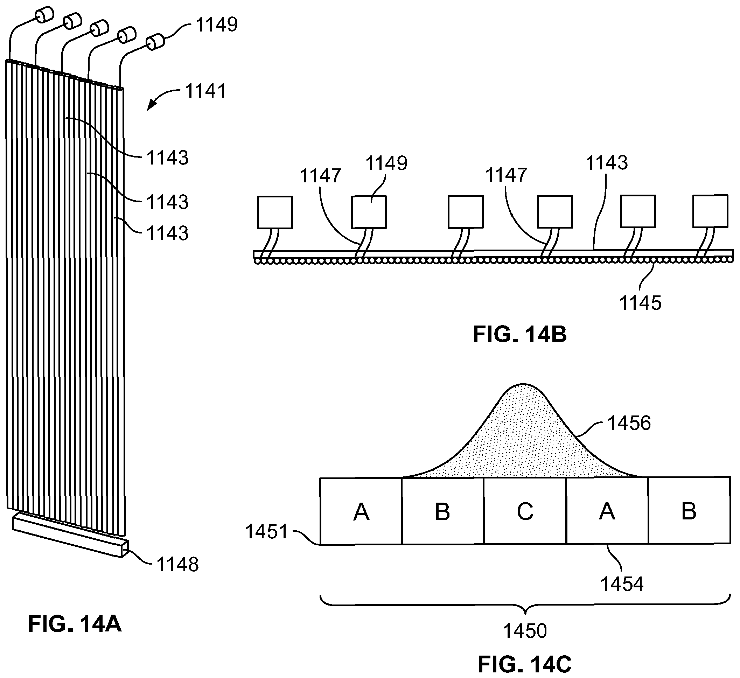

Referring now to FIGS. 14A and 14B, a segmented transmission detector, designated generally by numeral 1141, is shown for measuring a scan beam intensity profile of incident X rays 1143. Alignment of the Sc-WSF detector 1141 (used in transmission) with the plane of a scanning pencil beam presents a significant challenge when the TX detector is deployed for a mobile security system. FIG. 14B shows a cross section of a vertical Sc-WSF detector 1141 (otherwise referred to herein, when appropriate, as a "transmission detector" or "TX detector") with independent read-out of the fibers 1145 of the WSFs, provides the means to simultaneously measure both the transmitted intensity of each pixel and the linear distribution across the beam width to determine its centroid position. Fibers 1145 are routed in bundles 1147 to individual photo-detectors 1149 such as PMTs. The distribution of the intensity can extend out to obtain the forward scattered intensity, which contains useful information as to the scattering material, and gives a measure of the in-scattered radiation that is being counted as Transmission intensity.

The relative position of the detector plane and the plane of scanning X rays can be controlled automatically. The detector for this concept is shown schematically in FIG. 14A. A reflecting surface 1148 may be provided at an end of detector 1141 distal to photo-detectors 1149.

With a single data channel for a transmission signal, the spatial resolution along the traffic direction (transverse to a fan-shaped illuminating X-ray beam) is determined by the smaller of the following two dimensions: the width of the sensitive detector area or the beam size across the TX detector. (For heuristic purposes, the case of undersampling is not considered in this description.) Spatial resolution may be improved, however, by narrowing the sensitive detector area, as now described with reference to FIG. 14C. In accordance with embodiments of the present specification, the spatial resolution across the direction of traffic (along the detector line) is enhanced by employing multiple detectors of a detector array 1450 associated with a plurality of channels (A, B, C, in FIG. 14C) and interlacing their sensitive areas. The pitch of the interlace pattern depends on the beam width along the detector. Ideally the pitch (i.e., the spacing between two detectors 1451 and 1454 associated with a single channel "A") has to be large enough so that two detector segments of the same detection channel do not receive direct radiation from the beam at the same time. The beam intensity profile is depicted by numeral 1456. For practical purposes the requirement is not as stringent, since some amount of crosstalk between pixels is acceptable. The multiple, resulting images need to be interlaced, employing any method, including methods well-known in the art, to create one higher-resolution image. It should be noted that improvement of the spatial resolution at the detector comes at the expense of flux and is, thus, limited by signal-to-noise considerations.

Another configuration within the scope of the present specification include a combination of the vertical detector 1141 shown in FIG. 14A with horizontal road detector 1135 of FIG. 13B to form an L-shaped detector that is advantageously easily set up and aligned.

In yet another embodiment of the specification, a transmission detector array 1450 (regardless of geometrical orientation, whether vertical, horizontal, L-shaped, etc.) is segmented into a plurality of units; such as B, C and A of Fig 14C. As shown, the beam profile 1456 is symmetric with respect to B and A so that the ratio of the measured intensities is unity. If, for any reason, the alignment changes, that ratio changes dramatically. If the alignment skews as an illuminating X-ray pencil beam scans up and down, the change in the ratio of B/A measures the both the skewness and the lateral shift. Collected data can then be corrected for such a shift on a line-by-line basis.

Dual-Energy and Multi-Energy TX Detectors for Material Identification:

Separating the signals from front and back layers of scintillators allows the front layer to give a measure of the low-energy component of each pixel while the back layer gives a measure of the high-energy components. Putting a layer of absorbing material between the front and back scintillators is a standard way to enhance the difference between low and high energy components, and that is easily done with a Sc-WSF detector.

The Sc-WSF detector makes practical a dual-energy detector consisting of a layer of Sc-WSF, such as BaFCl-WSF, on top of a plastic scintillator detector; the BaFCl is sensitive to the low-energy X rays and not the high-energy X rays, while the plastic detector is sensitive to the high-energy X rays and very insensitive to low energy X rays.

An alternative and potentially more effective material discriminator can be made by using more than two independent layers of Sc-WSF, with separate readouts for each layer. A passive absorber, such as an appropriate thickness of copper, can be inserted after the top Sc-WSF to enhance dual energy application, as is practiced with segmented detectors. Alternatively, the middle scintillator can be used as an active absorbing layer. The measurement of three independent parameters allows one to get a measure of both the average atomic number of the traversed materials and the extent of beam hardening as well. The Sc-WSF can be further extended to obtain more than three energy values for each pixel, the limit being the statistical uncertainties, which increase with the number of components. Detector 1400 shown in FIG. 12 is an extreme example of such a detector.

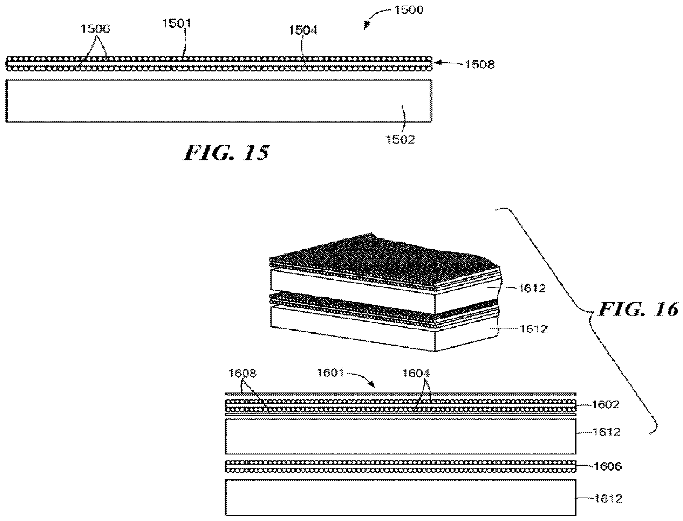

An important application of Dual-Energy TX is for X-ray personnel scanners at airport terminals. Providing TX images simultaneously with BX has proved useful for inspection. Adding dual-energy to the TX images has hitherto been impractical primarily because of size constraints imposed by conventional detectors. Sc-WSF eliminates those constraints and promises to significantly improve performance, since multiple detectors, with distinct energy sensitivities, may be stacked, as shown in FIG. 15, where a dual- (or multi-) energy detector 1500 includes an Sc-WSF detector 1508, sensitive to a lower energy component of incident X rays 1501, positioned in front of a slab of plastic scintillator 1502, which is sensitive to the higher energy X rays. Sc-WSF detector 1508 contains a scintillator 1504 read out by two layers of WS fibers 1506.

Compact Radiation Detector for Gamma and Neutron Radiation:

The Sc-WSF method makes practical a small, lightweight, inexpensive, monitor of neutrons and gamma rays 1601. BaFCl(Eu)-WSF is quite sensitive to gamma radiation while being insensitive to neutrons, while Li6F:ZnS(Ag)-WSF is insensitive to gamma rays and quite sensitive to detecting thermal neutrons. FIG. 16 shows a multi-layer "Dagwood" sandwich consisting of one or more layers 1602 of BaFCl(Eu), read out by a single photo-detector (not shown) via optical fibers 1604, and one or more layers 1606 of Li6F:ZnS(Ag)-WSF, read out by a second, independent, photo-detector (not shown), with the active elements occupying a thickness of no more than one or two centimeters. An appropriate layer of neutron moderator 1612, such as polyethylene, may be placed on either side of the Li6F:ZnS(Ag)-WSF to enhance the efficiency for detecting neutrons. Optically reflective foil 1608, such as aluminum foil, confines scintillation to respective detector regions.

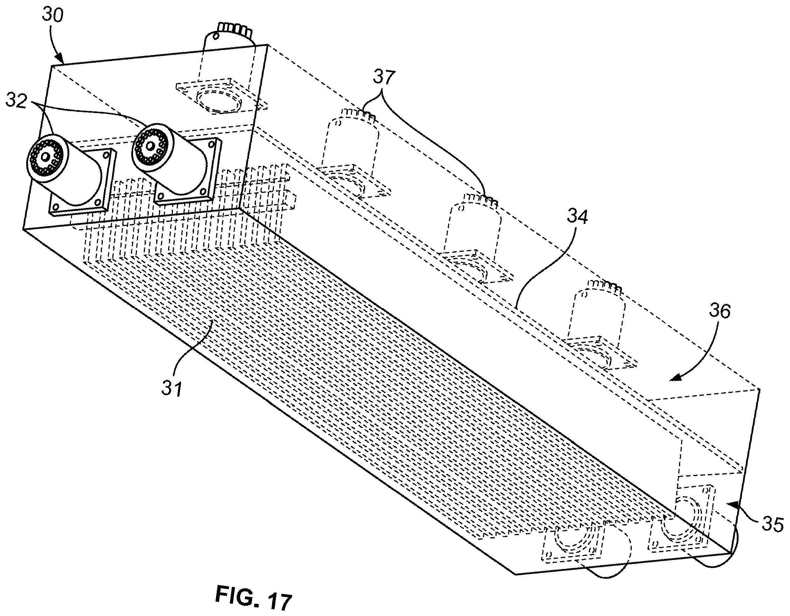

US Patent Application, Serial No. 13/163,854 (to Rothschild), entitled "Detector with Active Collimators," describes a backscatter detector module 30 that increases the depth of inspection by distinguishing scatter from the near and far field of inspected objects, as depicted in FIG. 17. The angle of a set of active collimating vanes 31 may either be adjusted once at the factory, or may be attached to any kind of electro-mechanical device provided to dynamically adjust them, depending on the type and/or distance of the object being scanned. The scintillation light from the collimating vanes is detected by one or more photo detectors (for example, by PMTs 32 located at the top and bottom of the front compartment of the detector). A rear compartment 36 of the detector is optically isolated from a front compartment 35 by a light baffle 34, and scintillation light from X rays detected in rear compartment 36 are collected by a second set of one or more photo-detectors (for example, PMTs 37 mounted on the rear face of the detector. The rear compartment may be lined with scintillating phosphor screen, for example, or, in other embodiments of the specification, may contain plastic or liquid scintillator.

A useful addition to a standard backscatter unit would be a "Venetian blind" collimator made of scintillator. The slats intercept radiation that does not enter directly through the gaps between the slats so that the box detectors preferentially detect deeper interior objects. The active collimators record the rejected radiation. The light from the active collimators is detected by PMTs, whose collection efficiency decreases rapidly as the gap between collimators decrease. Replacing the PMTs and scintillator vanes with vanes consisting of Sc-WSF detectors solves major shortcomings and makes venetian-blind collimators practical. First, light collection is independent of the gap width between vanes. Second, the active area of the PMTs or silicon photomultipliers used to collect the light from the active collimators is generally much smaller than the active area of needed PMTs, so that the cost of the photo-detectors is less. Third, the placement of the photo detector at the end of the WSF bundles is not critical to the efficiency of the light collection. Fourth, the signals from the WSFs from each slat can be processed independently, giving considerable scope for maximizing the information about the interior of the inspected object. Fifth, the light from the thin scintillator screens on the front and back of each vane can be collected by independent WSFs, which can significantly improve the depth discrimination.

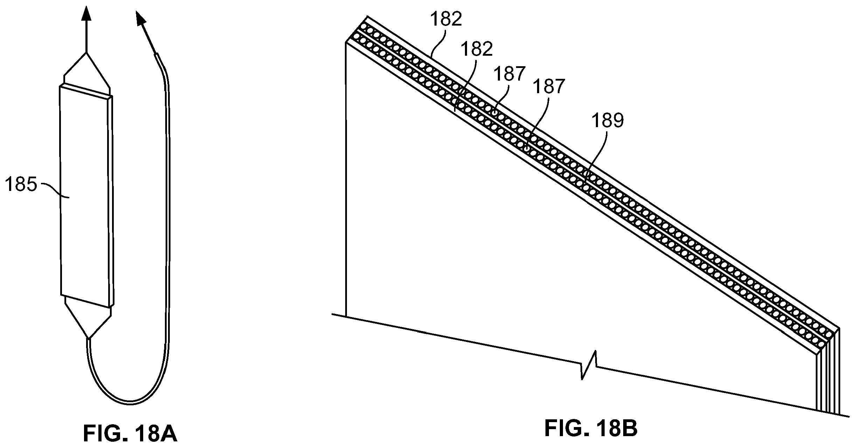

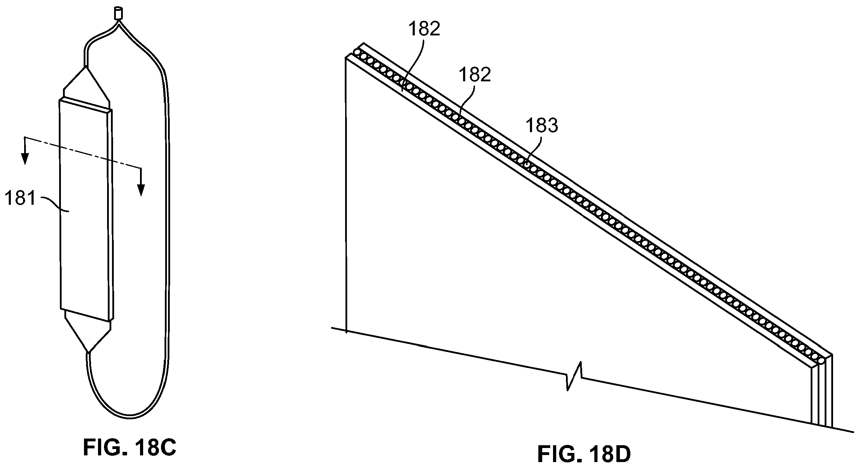

**FIGS. 18C** and 18D depict (in perspective and in cross section, respectively) an active WSF collimator 181 sensitive to X rays impinging from either side of the scintillator. Scintillation light from both scintillator regions 182 is coupled to photo-detectors via waveshifting optical fibers 183. FIGS. 18A and 18B show (in perspective and in cross section, respectively) an active WSF collimator 185 with independent readouts 187 separated by a light-tight X-ray absorber 189 to distinguish radiation striking each face. For example, each collimator 185 may consist, in one embodiment, of two layers of Sc-WSF detectors 182, each containing an areal density of 60mg BaFCl:Eu per cm2. The light-tight X-ray absorber 189 may consist of a thin layer of tin, which also provides structural support.

Detectors for Mini-Backscatter Inspection Systems:

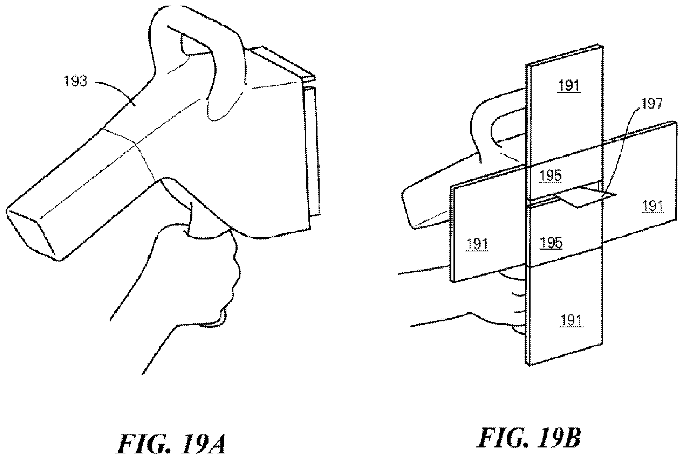

The thinness of Sc-WSF detectors provides a unique potential for applications in which low weight and power are drivers. Referring to FIGS. 19A and 19B, a hand-held imaging system 193 is an example of such an application. The power requirements, inspection time, and, quality of the image, are all affected by the solid angle of detection. A traditional detector with, for example, a cross-section of 10cm × 10cm (100 cm2), weighs about a half a kilogram. A 10-cm cube of Sc-WSF, weighing no more than twice as much, can be made of individual Sc-WSF 10 cm × 10 cm detectors, each less than 5mm thick, that can be unfolded to present a backscatter detection area of at least 2,000 cm2, a twenty-fold increase in this example. The additional detection coverage can make an order of magnitude improvement in the hand-held system's performance.

The thin profile of Sc-WSF detectors described herein provide for fitting contoured detectors into tight spaces. For example, detectors may be adapted for personnel scanners constrained to fit into constricted airport inspection spaces.

FIG. 19 shows an example in which four detectors 191 fold or slide out of hand-held scanner 193 to substantially increase the detection efficiency, especially for items concealed deeper in the object being inspected. Backscatter detectors 195 straddle irradiating beam 197. Back-scatter inspection of the underside of stationary vehicles:

The inspection of the underside of vehicles by a portable X-ray backscattering system presents special problems. The road clearance of cars is not more than 8" and can be as little as 6". Fixed inspection systems, such as portals, can place a detector in the ground, or, as described above, can be placed on the ground using Sc-WSF. Mobile under-vehicle inspection systems, however, which are needed for security in many areas, have never been developed. Inspectors rely on passive inspection tools such as mirrors and cameras, which miss contraband in the gas tank or are camouflaged to appear innocuous.

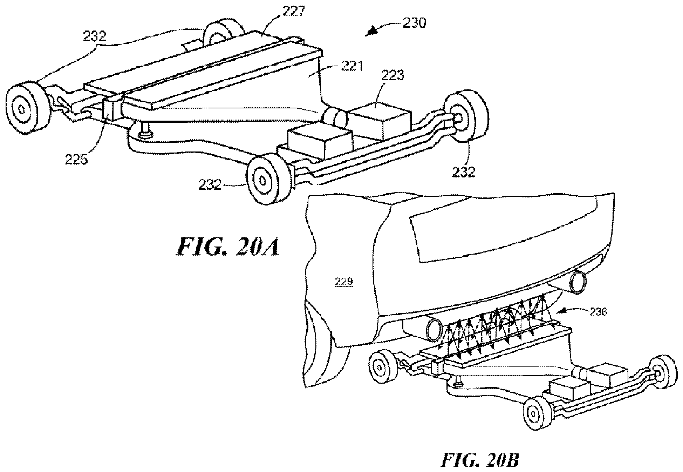

The Sc-WSF detectors make practical an X-ray backscatter system that is not more than 6" high. A sketch of a practical system is now described with reference to FIGS. 20A and 20B. The X-ray source consists of an electromagnetic scanner 221 of an electron beam across an anode. Electromagnetic scanner 221 is driven by electronics module 223. The X rays are collimated by a linear array of apertures 225 that span, for example, 30" of the underside in one pass. The Sc-WSF detectors 227 are mounted on each side of the X-ray tube so as detect X rays 236 backscattered from vehicle 229. Power supplies, pulse and image processors can be mounted appropriately. Chassis 234 of inspection unit 230 on wheels 232 may be adapted to be maneuvered under vehicle 229 by motor or manual control.

Mobile Transmission Inspection with L-Shaped Detector Array Segments:

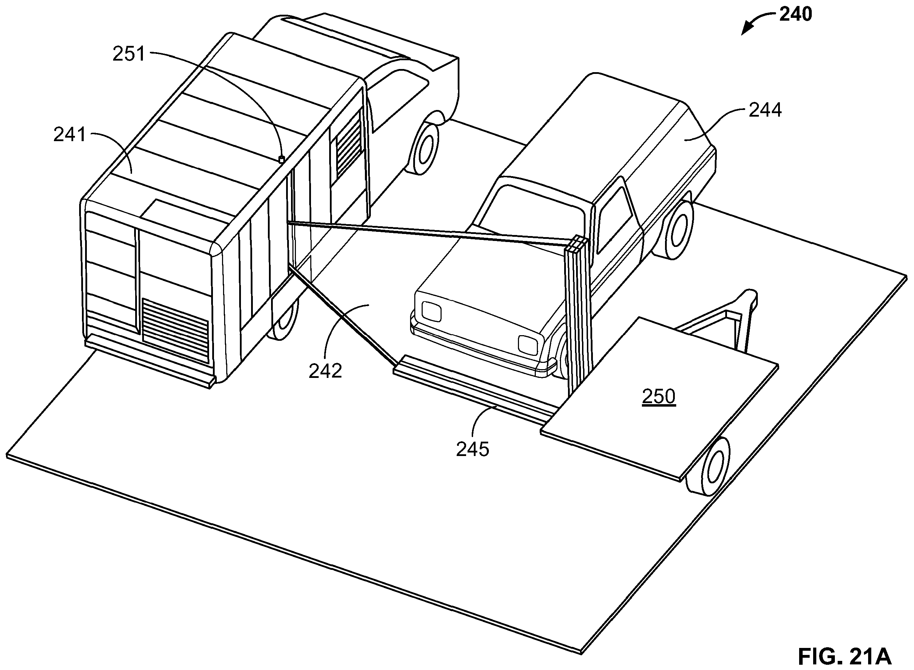

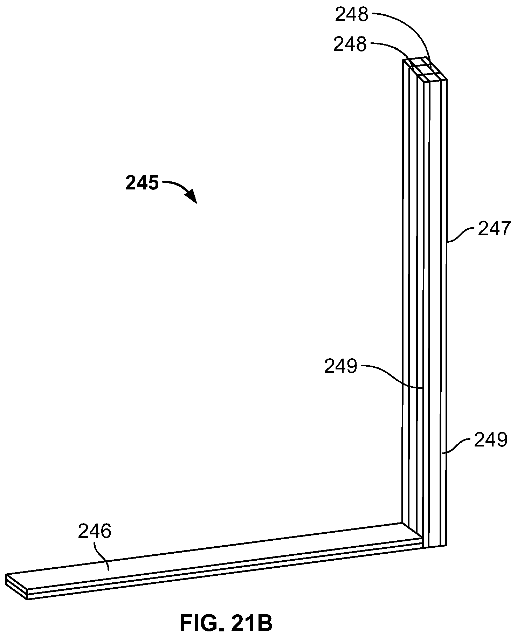

In accordance with another aspect of the present specification, a mobile inspection system, designated generally by numeral 240, is now described with reference to FIGS. 21A and 21B. A source of penetrating radiation (not shown, and described, herein, without limitation, in terms of X rays) is conveyed within a mobile inspection unit 241, which, typically, is capable of motion under its own power, although it may also be towed or otherwise transported, within the scope of the present specification. A beam 242 of penetrating radiation is emitted from mobile inspection unit 241, either as a swept pencil beam or as a fan beam, in either case emitted in the plane designated as representing beam 242 in FIG. 21A. Inspected object 244, which may be a vehicle as shown, or otherwise (such as hauled cargo), traverses beam 242 during the course of inspection, and, in the course of traversal, passes over an integral L-shaped detector unit 245, as now further described. Detector unit 245 has a horizontal segment 246 and an upright segment 247, as indicated in FIG. 21B.

Each of the horizontal and upright segments 246 and 247 of L-shaped detector unit 245 may be comprised of multiple parallel layers 249, providing for dual- or, more generally, multiple-, energy resolution of detected X rays, so as to provide material identification, as described above with reference to FIG. 12. Additionally, upright detector array segment 247 may have multiple detector segments 248 in a direction transverse to the direction of beam 242 and substantially along the direction of relative motion between inspected object 244 and beam 242 so as to provide an indication of skewness or lateral shift of the detectors with respect to the beam, as described above with reference to FIGS. 14A-14C. Integral L-shaped detector unit 245 may be conveyed to a site of inspection aboard mobile inspection unit 241 or on a towed, or otherwise accompanying, trailer 250, and may be assembled, in part, upon deployment at the inspection site. Supplemental alignment aids, such as alignment laser 251, may be employed in establishing proper position and orientation of detector unit 245 relative to mobile inspection unit 241 and beam 242.

Enhanced Resolution WSF Detectors:

In an embodiment, the present specification provides a system and method for enhancing the resolution of WSF detectors employed in an X-ray imaging system, and particularly in a flying spot X-ray imaging system. In an embodiment, an enhanced resolution WSF detector comprises at least a high resolution detection layer for detecting the intensities of incident radiation and a low resolution layer for detecting location of incidence radiation; thereby providing enhanced radiation detection.

In an embodiment, the enhanced resolution WSF detector of the present specification increases spatial sensitivity for X-ray detectors in an X-ray imaging system through the use of multiplexed WSF coupled to a multi-anode PMT. In an embodiment, the detector comprises multiple layers of WSF in order to determine both the high resolution content of the image by detecting the intensity captured by individual fibers, as well as low resolution mapping in order to determine a coarse location of the focal spot. In this way, a high resolution image is generated with a minimum of data individual channels, thus saving cost and complexity of the system.

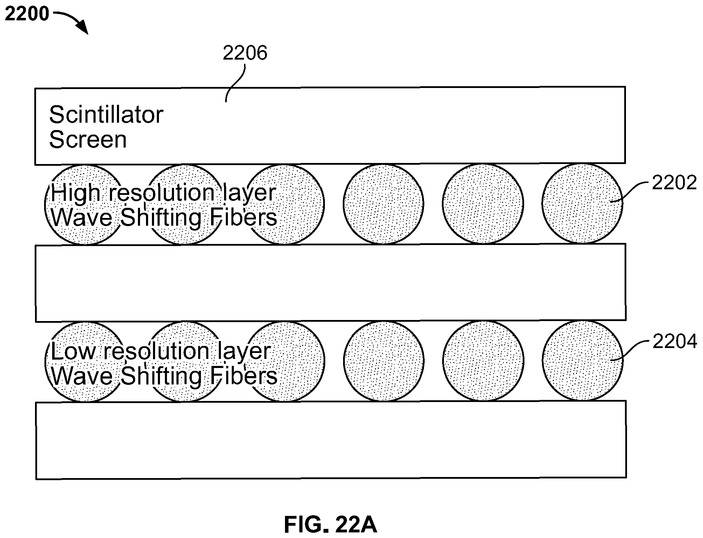

FIG. 22A illustrates a WSF detector with enhanced resolution, in accordance with an embodiment of the present specification and encompassed by the appended claims. In the embodiment, as shown in the figure, detector 2200 comprises a high resolution layer 2202 and low resolution layer 2204. In an embodiment, the high resolution layer 2202 has a width equal to the fiber width ranging from 0.25 mm to 2 mm and more specifically 1 mm. In an embodiment, the high resolution layer 2202 has a width equal to the maximum size of a flying spot X-ray beam used in conjunction with the detector, which in embodiments is less than 10 mm.

The low resolution layer 2204 comprises a plurality of parallel positioned fibers that are bundled from each of the low resolution regions of the detector 2200. In the embodiment, a position of an illumination beam spot i.e. the spatial resolution of the detector 2200 is determined by the signal detected in the low resolution layer 2204 of the detector. The signal intensity from the high resolution channels is subsequently placed in a correct spatial location using the information from the low resolution layer. The maximum intensity of the low resolution layer 2204 is used to identify the location of the flying spot X-ray beam on the detector.

Light absorbed in the high resolution fiber layer 2202 spreads, which degrades the spatial resolution of the WSF detector 2200. The spreading of light can be improved by utilizing a thin scintillator material 2206 as well as thin film deposited columnar materials which limit light scatter, coupled with the high resolution layer 2202, as shown in FIG. 22A. In an embodiment, Cesium Iodide is deposited as a thin film for limiting light scatter.

The spatial resolution of the detector 2200 is limited by the fiber diameter and spacing in the direction perpendicular to the fibers in the high resolution layer 2204. The spatial resolution in the orthogonal direction is limited by an illumination width of an incident fan beam of X rays. The fan beam width can be improved by using an X-ray source with a small focal spot size, and by using a narrow fan-beam collimator.

Hence, the spatial resolution of the WSF detector is determined by the fiber geometry of the high resolution layer, including spacing, shape and diameter of the fibers. In various embodiments, plastic wave-shifting optical fibers are made with diameters as low as 200 micro meters. By offsetting the fibers, the one dimensional spacing can further be reduced below 200 micro meters. In various embodiments, the high resolution layer comprises fibers having a diameter no greater than about 1 mm with no spacing between said adjacent fibers. Thus, in embodiments, the adjacent fibers are in physical contact with one another.

According to the embodiment encompassed by the claims, detectors for an X-ray imaging system comprise: a scintillation screen defining a detection region and at least one high resolution layer, optically coupled to the scintillation screen, comprising a first plurality of wavelength-shifting optical fibers. Each of the first plurality of wavelength-shifting optical fibers is defined by a first fiber radius and a first spacing between adjacent ones of the first plurality of wavelength-shifting optical fibers, wherein each of the first plurality of wavelength-shifting optical fibers extends through the detection region and under the scintillation screen, and wherein the first plurality of wavelength-shifting optical fibers is configured to receive radiation and generate signals; at least one low resolution layer comprising a second plurality of wavelength-shifting optical fibers wherein each of the second plurality of wavelength-shifting optical fibers is defined by a second fiber radius and a second spacing between adjacent ones of the second plurality of wavelength-shifting optical fibers, and wherein at least one of the second fiber radius is larger than the first fiber radius or the second spacing is greater than the first spacing, and wherein the second plurality of wavelength-shifting optical fibers is configured to receive the radiation that passes through the at least one high resolution layer and generate signals; and a segmented multi-channel photomultiplier tube configured to receive signals obtained from the at least one low resolution layer and to receive signals obtained from the at least one high resolution layer.

In some embodiments of the present specification, detectors for an X-ray imaging system comprise: at least one high resolution layer comprising a first plurality of wavelength-shifting optical fibers wherein each of the first plurality of wavelength-shifting optical fibers is defined by a first fiber radius and a first spacing between adjacent ones of the first plurality of wavelength-shifting optical fibers, wherein each of the first plurality of wavelength-shifting optical fibers extends through the detection region and under the scintillation screen, and wherein the first plurality of wavelength-shifting optical fibers is coated with scintillation material and is configured to receive radiation and generate signals; at least one low resolution layer comprising a second plurality of wavelength-shifting optical fibers wherein each of the second plurality of wavelength-shifting optical fibers is defined by a second fiber radius and a second spacing between adjacent ones of the second plurality of wavelength-shifting optical fibers, and wherein at least one of the second fiber radius is larger than the first fiber radius or the second spacing is greater than the first spacing, and wherein the second plurality of wavelength-shifting optical fibers is coated with scintillation material and is configured to receive the radiation that passes through the at least one high resolution layer and generate signals; and a segmented multi-channel photomultiplier tube configured to receive signals obtained from the at least one low resolution layer and to receive signals obtained from the at least one high resolution layer.

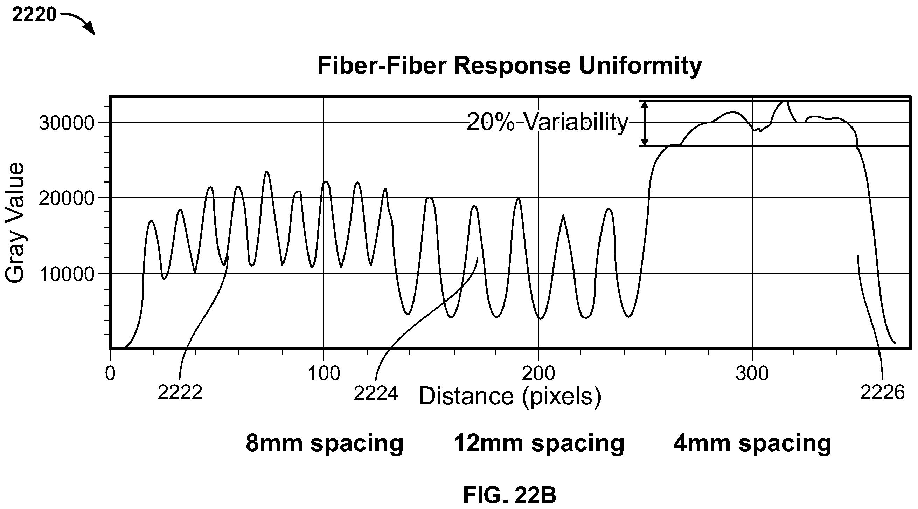

FIG. 22B illustrates a graph 2220 depicting the fiber response uniformity of the detector shown in FIG. 22A. The graph 2220 shows the detector response 2222, 2224 and 2226 when the fibers are separated by a distance of 8mm, 12mm and 4mm respectively. As can be seen, variability in the response 2226 when the WSF fibers of the detector are separated by a distance of 4mm is minimum, being approximately 20%.

In an embodiment, the high resolution layer 2202 comprises a set of 8 (only 5 shown in FIG. 22A) high resolution fibers which are configured to occupy 8 regions of the detector. In embodiments, the number of fibers is limited by a number of elements in a PMT coupled with said fibers, which must include the high and low resolution detection capabilities. Segmented PMT's typically have 16 elements, so in embodiments, a maximum of 15 elements may be dedicated to the high resolution fibers and one to the low fiber.

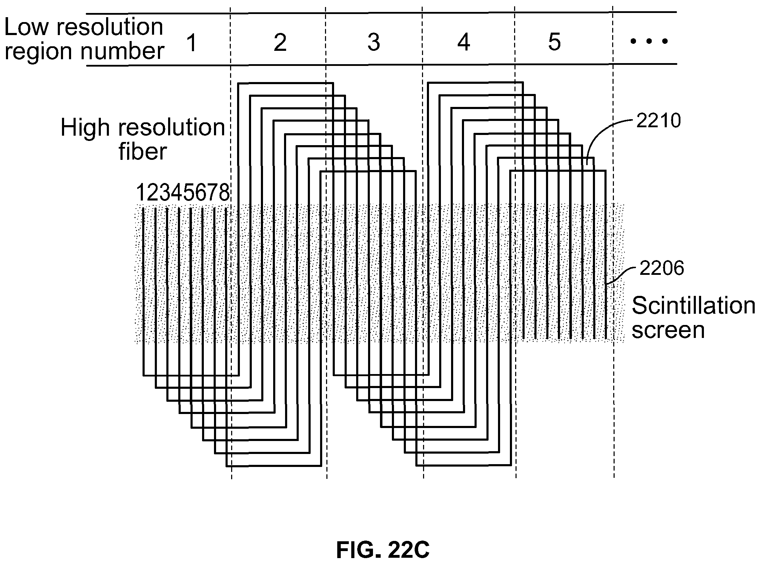

FIG. 22C illustrates a high resolution fiber layout, in accordance with an embodiment of the present specification. FIG. 22C also shows the addressing of detector signals coupled with a segmented PMT (not shown in the FIG.). In the case where the PMT has 16 elements, 8 channels are dedicated to the high resolution fibers and 8 are dedicated to low resolution fibers. In order to obtain a beam placement position for obtaining the intensity distribution at high resolution in the detector 2200, a number of high resolution fibers for each region are required. In an embodiment as shown in FIG. 22C, high resolution fibers comprise a series of WS fibers 2210 placed parallel to each other with minimum or no spacing, where each WS fiber 2210 extends through, out, and back into a detection region under a scintillation screen 2206.This configuration of fibers provides a dense detection area without having too many individual WSFs entering a PMT coupled with the WSF detector. In an embodiment, as shown in FIG. 22C due to repeated usage of 8 WS fibers in and out of the detection region in a serpentine configuration allows for a single WS fiber 2210 to detect multiple instances of impinging light across both the width and breadth of the WSF detector. Hence, this configuration makes the WSF detector more sensitive to intensity of incident radiation in comparison to a detector comprising WSFs laid out in a straight configuration. The high resolution fibers shown in FIG. 22C better detects intensity due to the dense WS coverage provided by using a small number (eight) of looping fibers 2210. In an embodiment, the high resolution layer of WSF comprises fifteen fibers placed in close contact and parallel to each other, to provide in a loop back configuration as shown in FIG. 22C. In various embodiments, a maximum distance between the fibers is 1mm.

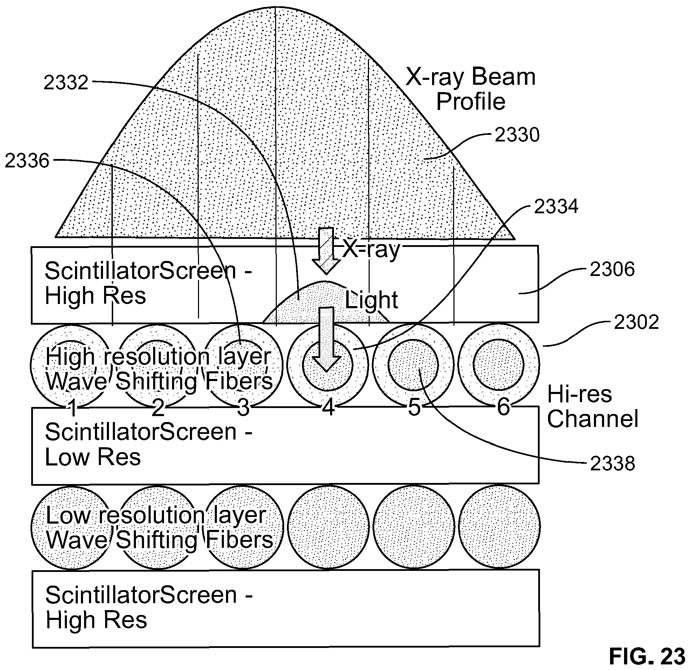

FIG. 23 illustrates X-ray absorption and light collection in a high resolution layer of a WSF detector, in accordance with an embodiment of the present specification. Incident X ray beam 2330 is absorbed by a scintillator screen 2306 of the WSF detector 2300. Following X-ray absorption, visible scintillation light propagates in the screen 2306 and is scattered or absorbed. As the light spreads due to scattering in the scintillator layer 2306, it is distributed over a spatial area and as a result, a single X-ray event may couple light intensity to multiple fibers. As seen in the figure, the green light plume 2332 spreads beyond the region of fiber 4, 2334 and into fiber 3, 2336 and fiber 5, 2338. In an embodiment, high resolution fibers detection regions may be positioned in predefined areas of a WSF detector array so that an incident X ray beam may also be positioned in said areas only, for improving detection efficiency of the WSF detector.

The density of the scintillators for each layer of the detector 2300 is tuned so that the detector achieves a high resolution in a front scintillator but would allow enough X-ray through to affect detection of both high energy and low energy radiation.

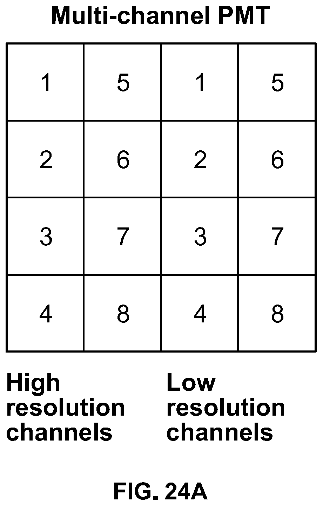

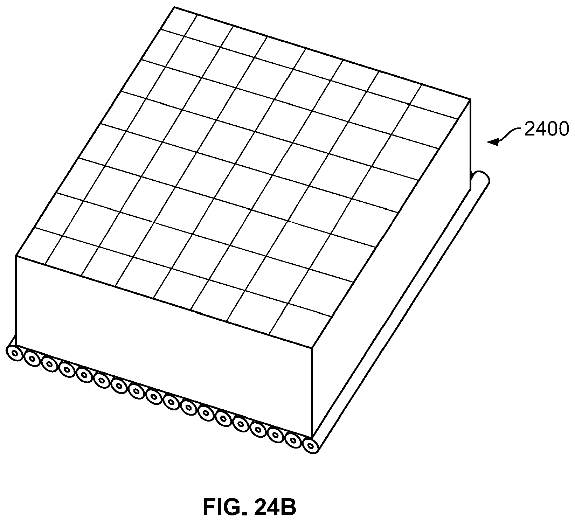

As described above, the ends of the WSF are bundled and optically coupled to at least one photodetector. In an embodiment the detector resolution is enhanced by detecting the signal intensity for individual WSF fibers with a multi-channel PMT. In an embodiment, in order to reduce the number of channels to a manageable size, individual read-outs corresponding to each WSF fiber are multiplexed between high and low resolution layers of the WSF detector. FIG. 24A illustrates a 16-channel PMT coupling signals obtained from all high resolution fibers and low resolution fibers of the detector illustrated in FIG. 22A, in accordance with an embodiment of the present specification. In various embodiments, the multi-layer detector 2200 multiplexes the fibers/channels and provides a detector readout that is cost-effective. In an embodiment, commercially available segmented PMT's may be used. FIG. 24B is a diagrammatical representation of a 64-channel multi-anode PMT assembly 2400 that may be used for reading the light signals captured by the WSF detector of the present specification.

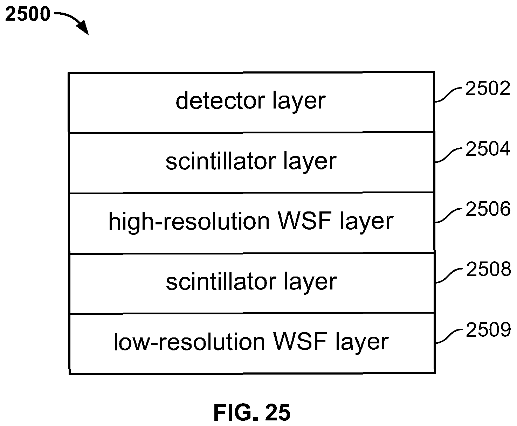

FIG. 25 is a block diagram illustrating the layers of an exemplary multi-layer high-resolution detector, in accordance with an embodiment of the present specification. Detector 2500 comprises a first horizontal detector layer 2502, a second scintillator layer 2504, a third high-resolution WSF layer comprising individual fibers coupled to PMT anodes 2506, a fourth scintillator layer 2508, and a fifth layer 2509 comprising low-resolution WSF groups of fibers coupled to PMT anodes. In an embodiment, one or more scintillator filters are embedded in the multi-layer WSF detector, in order to separate materials in the image data obtained via the detector. In an embodiment, firstly the fibers are placed in a desired configuration and then scintillator is molded around the fiber configuration. In another embodiment, each fiber is first coated with scintillator and then placed in the desired position in the WSF detector.

Enhanced Resolution WSF Detector Panels:

In an embodiment, the present specification provides a detector panel comprising WSF detectors. The detector panel is designed for placement at any position relative to a portable/handheld scanner. In an embodiment where said detector panel is placed in the direct beam of an X-ray source, the detector panel acts as a transmission detector.

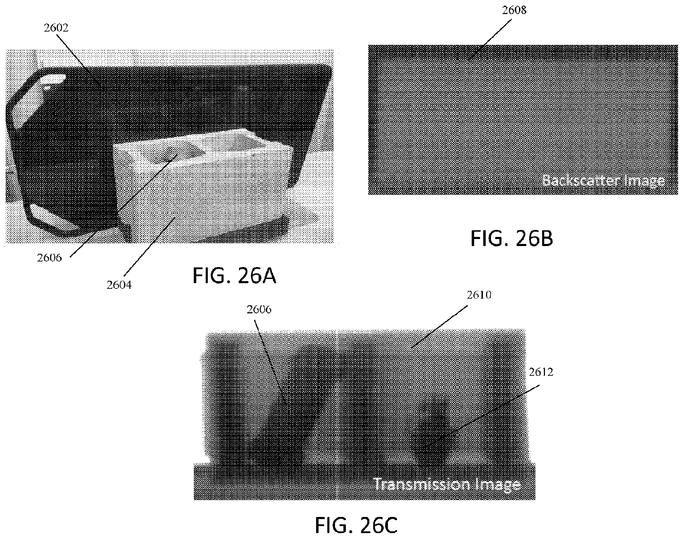

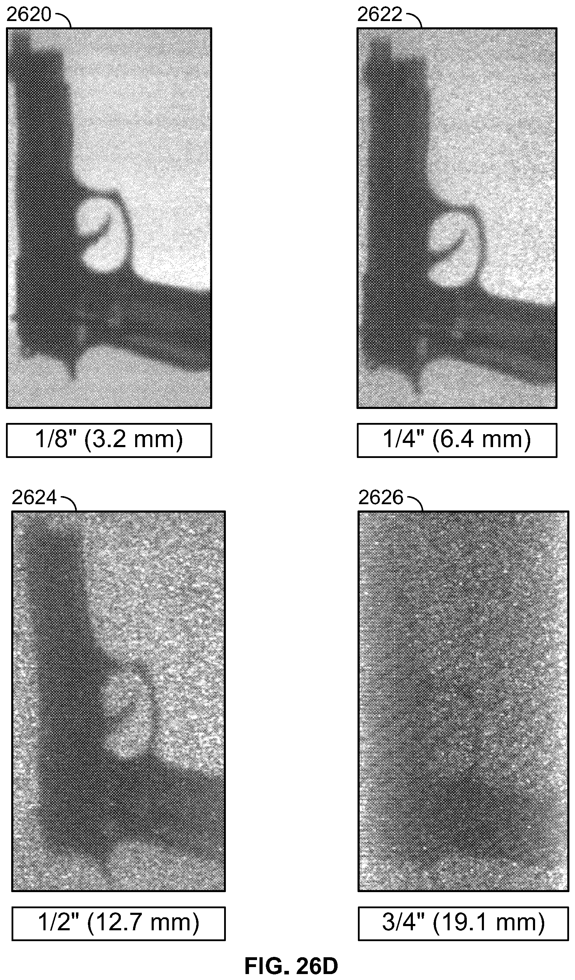

FIG. 26A illustrates a detector panel placed in direct beam of scanning radiations emitted by a small portable scanner being used to scan an object, in accordance with an embodiment of the present specification. As shown, detector panel 2602 is placed behind a concrete block 2604 which is being scanned by a portable scanner comprising an X-ray source (not shown in the figure) such that the detector panel 2602 is placed in a direct beam path of the radiation being emitted by said source. The concrete block 2604 contains a steel pipe bomb 2606 (partially visible in FIG. 26A) and a hand grenade (not visible in FIG. 26A). FIG. 26B illustrates a backscatter image obtained by the scanner of FIG. 26A, in accordance with an embodiment of the present specification. A backscatter image 2608 obtained by said hand held scanner, which is obtained using the in-built detectors of said scanner does not show either the steel pipe bomb 2606 or the hand grandee contained within the concrete block 2604. FIG. 26C illustrates a transmission image obtained by a built-in detector of the scanner of FIG. 26A by using the detector panel 2602 as shown in FIG. 26A, in accordance with an embodiment of the present specification. As can be seen in FIG. 26C, the transmission image 2610 clearly shows a hand grenade 2612 and the steel pipe bomb 2606 contained within the concrete block 2604. The spatial resolution of the transmission image 2610 is governed by the scanning beam spot size, however, the beam penetration and SNR is greatly enhanced as compared to the backscatter image 2608. FIG. 26D illustrates transmission images of a gun placed behind steel walls of different thickness obtained by using the detector panel shown in FIG. 26A. Images 2620, 2622, 2624, 2626 illustrates the images of a gun placed behind 3.2mm, 6.4mm,12.7mm, and 19.1mm of steel respectively.

With the use of detector panels along with portable/hand held scanners, as shown in FIGS. 26A, a challenge is that there is no pre-established physical configuration between the detector and the scanning source. If the position of the source relative to the detector is known/fixed, the location of impurities and irregularities in the detector may be fixed, and hence, any detected data could be automatically corrected for said irregularities. Specifically, the gain could be corrected (increased/decreased) to account for spots or lines due to issues in manufacturing of the scanner/detector. However, with the use of the detector panel as described in the present specification, the relative configuration of the detector panel and the scanning source is changeable, making it difficult to predict precisely the location of non-uniformities in the scanning image. Hence, the non-uniformities that are inherent to the detector response cannot be corrected using known gain correction methods. Without gain calibration the signals received by using the scanner and detector as shown in FIG. 26A will be raw and may include defects such as, but not limited to: gain non-uniformity due to variations in the X-ray absorption in scintillator; non uniformity in the scintillator light production and propagation; and non-uniformity in light collection across the area of detector.

The challenge, therefore, is to create a detector panel where X-ray spot generates same amount of light at a PMT corresponding to any spot that X-ray hits the detector panel so that no gain correction is required. The more uniform the response, the lower the variability. With the use of conventional fixed X-ray source detector configurations, a variability ranging from 30% to 40% may be tolerated. However, for handheld scanner and detector configurations, a variability of 10% or less is required.

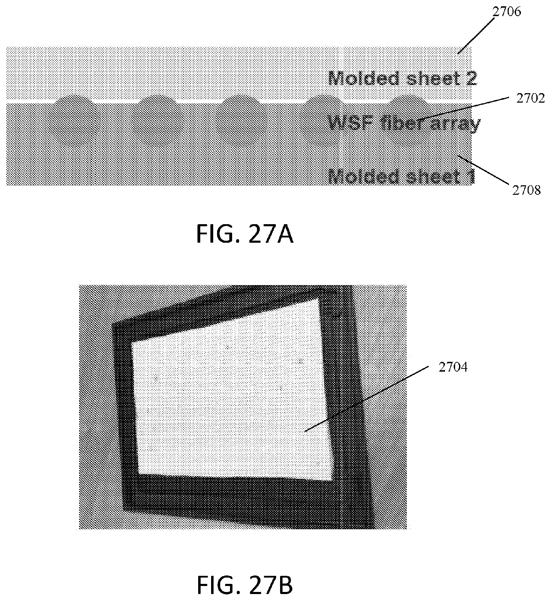

FIG. 27A illustrates a diagrammatical representation of a WSF detector panel, in accordance with another embodiment of the present specification. FIG. 27B illustrates the WSF detector panel of FIG. 27A, in accordance with an embodiment of the present specification. Referring to FIGS. 27A and 27B, a plurality of WSF fibers 2702 are held together at a predefined distance forming a detector panel 2704 by molded sheets of a transparent, flexible plastic binder 2706, 2708 with scintillator powder embedded. In some embodiments, the transparent, flexible plastic binder 2706, 2708 is silicone. In some embodiments, the transparent, flexible plastic binder 2706, 2708 is polyvinyl butyral (PVB) mixed with a plasticizer. In an embodiment, a spacing of 3 mm is maintained between the fibers 2702 by adjusting the scintillator powder concentration. A spacing of 3 mm is used as the variability in light intensity across the detector panel disappears at sizes greater than 4mm, as shown in FIG 22B. As the powder concentration in the detector panel decreases, the light is able to travel further, providing a more uniform response. In an embodiment, the ends of fibers 2702 are bundled into PMTs and may be read out from one or both ends, as shown in FIG. 8 above. The detector shown in FIGS. 27A, 27B is easy to manufacture, and minimizes the number of WSF fibers required to obtain a detector of a desired area. The detector also provides a uniform coupling of light in associated PMT leading to a signal detection.

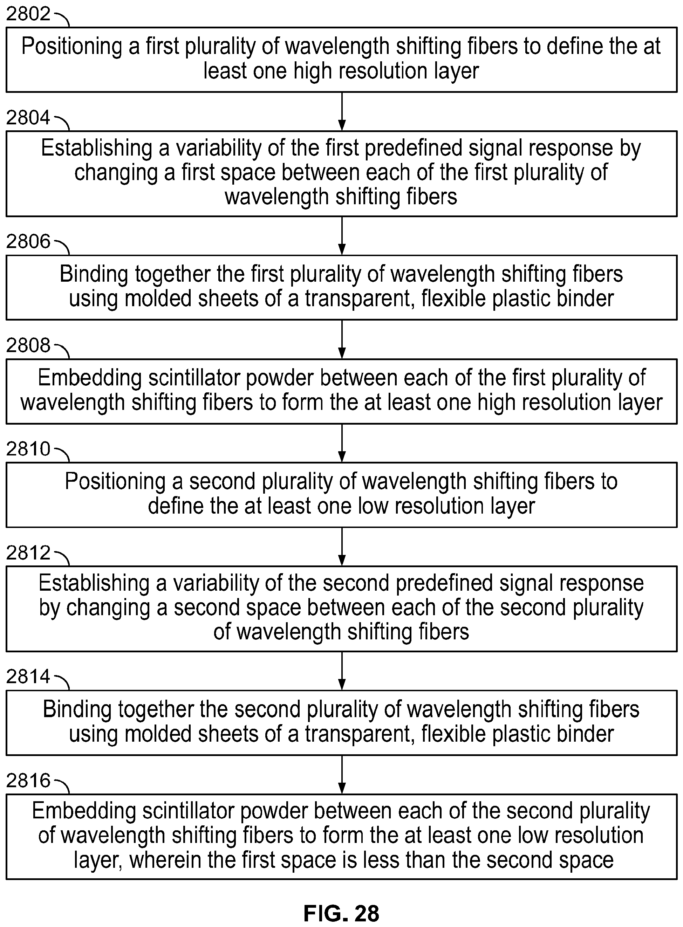

FIG. 28 is a flowchart illustrating the steps in a method of forming a detector with at least one high resolution layer and at least one low resolution layer, wherein the at least one high resolution layer has a first predefined signal response and wherein the at least one low resolution layer has a second predefined signal response, in accordance with some embodiments of the present specification. In some embodiments, the method comprises: at step 2802, positioning a first plurality of wavelength shifting fibers to define the at least one high resolution layer; at step 2804, establishing a variability of the first predefined signal response by changing a first space between each of the first plurality of wavelength shifting fibers; at step 2806, binding together the first plurality of wavelength shifting fibers using molded sheets of a transparent, flexible plastic binder; at step 2808, embedding scintillator powder between each of the first plurality of wavelength shifting fibers to form the at least one high resolution layer; at step 2810, positioning a second plurality of wavelength shifting fibers to define the at least one low resolution layer; at step 2812, establishing a variability of the second predefined signal response by changing a second space between each of the second plurality of wavelength shifting fibers; at step 2814, binding together the second plurality of wavelength shifting fibers using molded sheets of a transparent, flexible plastic binder; and, at step 2816, embedding scintillator powder between each of the second plurality of wavelength shifting fibers to form the at least one low resolution layer, wherein the first space is less than the second space.

The above examples are merely illustrative of the many applications of the system and method of present specification. Although only a few embodiments of the present specification have been described herein, it should be understood that the present specification might be embodied in many other specific forms without departing from the scope of the specification. Therefore, the present examples and embodiments are to be considered as illustrative and not restrictive, and the specification may be modified within the scope of the appended claims.

**Claims**

1. A detector (2200) for an X-ray imaging system, the detector comprising:

   a scintillator screen (2206) defining a detection region;
   at least one high resolution layer comprising a plurality of high-resolution wavelength-shifting optical fibers (2210) placed parallel to each other, wherein each of the plurality of high-resolution wavelength-shifting optical fibers extends through and out of the detection region and loops back into the detection region under the scintillation screen (2206) covering the high-resolution wavelength-shifting optical fibers, wherein each of the plurality of high-resolution wavelength-shifting optical fibers occupies a distinct region of the detector;
   at least one low resolution layer positioned under the high resolution layer and comprising a plurality of low-resolution regions having a plurality of low-resolution optical fibers (2204) laid out in a parallel configuration, wherein each of the plurality of low-resolution optical fibers is configured to shift received wavelengths; and
   a segmented multi-channel photomultiplier tube, PMT, for coupling signals obtained from the high-resolution fibers and the low-resolution fibers and for generating:

      first detector signals corresponding to the signals obtained from the high-resolution fibers and generated in response to light output by the scintillator screen, and
      second detector signals corresponding to the signals obtained from the low-resolution fibers and generated in response to radiation that passes through the at least one high resolution layer, wherein the first detector signals are indicative of intensities of X-ray radiation incident on the scintillator screen (2206) and the second detector signals are indicative of a spatial location of the incident X-ray radiation;
      wherein the detector is configured to:
      determine the spatial location of the incident X-ray radiation based on a maximum intensity of the second detector signals and spatially locate the intensities of the first detector signals based on the determined spatial location of the incident X-ray radiation.

2. The detector of claim 1 wherein each of the plurality of high-resolution fibers has a radius ranging from 0.2 mm to 2 mm.

3. The detector of claim 1, wherein the plurality of low-resolution regions comprises low resolution fibers with a radius ranging from 1 mm to 3 mm.

4. The detector of any preceding claim wherein the PMT comprises 8 to 16 channels.

5. The detector of any preceding claim wherein the scintillation screen (2206) is optically coupled to the at least one high resolution layer.

6. The detector of any preceding claim wherein each fiber of the plurality of high-resolution fibers and each fiber of the plurality of low-resolution fibers are made of plastic.

7. The detector of claim 1 wherein a diameter of each fiber of the plurality of high-resolution fibers and each of the plurality of low-resolution fibers is less than 200 micrometers.

8. The detector of any preceding claim wherein each fiber of the plurality of high-resolution fibers and each fiber of the plurality of low-resolution fibers are coated with a scintillating material.

9. The detector of claim 1 further comprising a scintillator layer positioned between the at least one high resolution layer and the at least one low resolution layer.

10. The detector of claim 1 further comprising one or more scintillator filters embedded in at least one of the at least one high resolution layer or the at least one low resolution layer.

11. The detector of claim 1, wherein at least one of a radius of the low-resolution fiber is greater than a radius of the high-resolution fiber or a spacing between the low-resolution fibers is greater than a spacing between the high-resolution fibers.

12. The detector of claim 11, wherein the high-resolution layer has a width equal to a maximum size of a flying spot X-ray beam incident on the scintillator screen (2206).

**Patentansprüche**

1. Ein Detektor (2200) für ein Röntgenbildgebungssystem, wobei der Detektor Folgendes beinhaltet:

   einen Szintillatorschirm (2206), der einen Detektionsbereich definiert;
   mindestens eine hochauflösende Schicht, die eine Vielzahl von hochauflösenden wellenlängenverschiebenden optischen Fasern (2210) beinhaltet, die parallel zueinander platziert sind, wobei sich jede der Vielzahl von hochauflösenden wellenlängenverschiebenden optischen Fasern durch den Detektionsbereich und aus diesem heraus erstreckt und unter dem Szintillationsschirm (2206), der die hochauflösenden wellenlängenverschiebenden optischen Fasern bedeckt, zurück in den Detektionsbereich schleift, wobei jede der Vielzahl von hochauflösenden wellenlängenverschiebenden optischen Fasern einen bestimmten Bereich des Detektors einnimmt;
   mindestens eine niedrigauflösende Schicht, die unter der hochauflösenden Schicht positioniert ist und eine Vielzahl von niedrigauflösenden Bereichen beinhaltet, die eine Vielzahl von niedrigauflösenden optischen Fasern (2204) aufweisen, die in einer parallelen Konfiguration angeordnet sind, wobei jede der Vielzahl von niedrigauflösenden optischen Fasern konfiguriert ist, um empfangene Wellenlängen zu verschieben; und
   eine segmentierte Mehrkanal-Photomultiplierröhre, PMT, zum Koppeln von Signalen, die von den hochauflösenden Fasern und den niedrigauflösenden Fasern erhalten werden, und zum Erzeugen von Folgendem:

   ersten Detektorsignalen, die den Signalen entsprechen, die von den hochauflösenden Fasern erhalten werden, und als Reaktion auf Lichtausgabe durch den Szintillatorschirm erzeugt werden, und
   zweiten Detektorsignalen, die den Signalen entsprechen, die von den niedrigauflösenden Fasern erhalten werden, und als Reaktion auf Strahlung erzeugt werden, die durch die mindestens eine hochauflösende Schicht verläuft, wobei die ersten Detektorsignale Intensitäten von Röntgenstrahlung angeben, die auf den Szintillatorschirm (2206) einfällt, und die zweiten Detektorsignale eine räumliche Lage der einfallenden Röntgenstrahlung angeben;

   wobei der Detektor für Folgendes konfiguriert ist:
   Bestimmen der räumlichen Lage der einfallenden Röntgenstrahlung basierend auf einer maximalen Intensität der zweiten Detektorsignale und räumliches Lokalisieren der Intensitäten der ersten Detektorsignale basierend auf der bestimmten räumlichen Lage der einfallenden Röntgenstrahlung.

2. Detektor gemäß Anspruch 1, wobei jede der Vielzahl von hochauflösenden Fasern einen Radius im Bereich von 0,2 mm bis 2 mm aufweist.

3. Detektor gemäß Anspruch 1, wobei die Vielzahl von niedrigauflösenden Bereichen niedrigauflösende Fasern mit einem Radius im Bereich von 1 mm bis 3 mm beinhaltet.

4. Detektor gemäß einem der vorhergehenden Ansprüche, wobei die PMT 8 bis 16 Kanäle beinhaltet.

5. Detektor gemäß einem der vorhergehenden Ansprüche, wobei der Szintillationsschirm (2206) optisch an die mindestens eine hochauflösende Schicht gekoppelt ist.

6. Detektor gemäß einem der vorhergehenden Ansprüche, wobei jede Faser der Vielzahl von hochauflösenden Fasern und jede Faser der Vielzahl von niedrigauflösenden Fasern aus Kunststoff hergestellt sind.

7. Detektor gemäß Anspruch 1, wobei ein Durchmesser jeder Faser der Vielzahl von hochauflösenden Fasern und jeder der Vielzahl von niedrigauflösenden Fasern weniger als 200 Mikrometer beträgt.

8. Detektor gemäß einem der vorhergehenden Ansprüche, wobei jede Faser der Vielzahl von hochauflösenden Fasern und jede Faser der Vielzahl von niedrigauflösenden Fasern mit einem Szintillationsmaterial beschichtet sind.

9. Detektor gemäß Anspruch 1, der ferner eine Szintillatorschicht beinhaltet, die zwischen der mindestens einen hochauflösenden Schicht und der mindestens einen niedrigauflösenden Schicht positioniert ist.

10. Detektor gemäß Anspruch 1, der ferner ein oder mehrere Szintillatorfilter beinhaltet, die in mindestens eine der mindestens einen hochauflösenden Schicht oder der mindestens einen niedrigauflösenden Schicht eingebettet sind.

11. Detektor gemäß Anspruch 1, wobei mindestens einer von einem Radius der niedrigauflösenden Faser größer als ein Radius der hochauflösenden Faser ist oder ein Abstand zwischen den niedrigauflösenden Fasern größer als ein Abstand zwischen den hochauflösenden Fasern ist.

12. Detektor gemäß Anspruch 11, wobei die hochauflösende Schicht eine Breite aufweist, die gleich einer maximalen Größe eines Flying-Spot-Röntgenstrahls ist, der auf den Szintillatorschirm (2206) einfällt.


**Revendications**

1. Un détecteur (2200) pour un système d'imagerie par rayons X, le détecteur comprenant :

   un écran de scintillateur (2206) définissant une région de détection ;
   au moins une couche à haute résolution comprenant une pluralité de fibres optiques à décalage de longueur d'onde à haute résolution (2210) placées parallèlement les unes aux autres, chaque fibre de la pluralité de fibres optiques à décalage de longueur d'onde à haute résolution s'étendant à travers et hors de la région de détection et revenant en boucle dans la région de détection sous l'écran de scintillation (2206) couvrant les fibres optiques à décalage de longueur d'onde à haute résolution, chaque fibre de la pluralité de fibres optiques à décalage de longueur d'onde à haute résolution occupant une région distincte du détecteur ;
   au moins une couche à basse résolution positionnée sous la couche à haute résolution et comprenant une pluralité de régions à basse résolution ayant une pluralité de fibres optiques à basse résolution (2204) disposées selon une configuration parallèle, chaque fibre de la pluralité de fibres optiques à basse résolution étant configurée pour décaler des longueurs d'onde reçues ; et
   un tube photomultiplicateur, PMT, multicanal segmenté pour le couplage de signaux obtenus à partir des fibres à haute résolution et des fibres à basse résolution et pour la génération :

      de premiers signaux de détecteur correspondant aux signaux obtenus à partir des fibres à haute résolution et générés en réponse à de la lumière émise en sortie par l'écran de scintillateur, et
      de deuxièmes signaux de détecteur correspondant aux signaux obtenus à partir des fibres à basse résolution et générés en réponse à un rayonnement qui passe à travers l'au moins une couche à haute résolution, les premiers signaux de détecteur étant indicatifs d'intensités de rayonnement de rayons X incident sur l'écran de scintillateur (2206) et les deuxièmes signaux de détecteur étant indicatifs d'une localisation spatiale du rayonnement de rayons X incident ;

   le détecteur étant configuré pour :
   déterminer la localisation spatiale du rayonnement de rayons X incident sur la base d'une intensité maximale des deuxièmes signaux de détecteur et localiser spatialement les intensités des premiers signaux de détecteur sur la base de la localisation spatiale déterminée du rayonnement de rayons X incident.

2. Le détecteur de la revendication 1 dans lequel chaque fibre de la pluralité de fibres à haute résolution a un rayon allant de 0,2 mm à 2 mm.

3. Le détecteur de la revendication 1, dans lequel la pluralité de régions à basse résolution comprend des fibres à basse résolution présentant un rayon allant de 1 mm à 3 mm.

4. Le détecteur de n'importe quelle revendication précédente dans lequel le PMT comprend de 8 à 16 canaux.

5. Le détecteur de n'importe quelle revendication précédente dans lequel l'écran de scintillation (2206) est couplé optiquement à l'au moins une couche à haute résolution.

**6.** Le détecteur de n'importe quelle revendication précédente dans lequel chaque fibre de la pluralité de fibres à haute résolution et chaque fibre de la pluralité de fibres à basse résolution sont faites de plastique.

**7.** Le détecteur de la revendication 1 dans lequel un diamètre de chaque fibre de la pluralité de fibres à haute résolution et de chaque fibre de la pluralité de fibres à basse résolution est inférieur à 200 micromètres.

**8.** Le détecteur de n'importe quelle revendication précédente dans lequel chaque fibre de la pluralité de fibres à haute résolution et chaque fibre de la pluralité de fibres à basse résolution sont revêtues d'un matériau scintillant.

**9.** Le détecteur de la revendication 1 comprenant en outre une couche de scintillateur positionnée entre l'au moins une couche à haute résolution et l'au moins une couche à basse résolution.

**10.** Le détecteur de la revendication 1 comprenant en outre un ou plusieurs filtres de scintillateur incorporés dans au moins l'une de l'au moins une couche à haute résolution ou de l'au moins une couche à basse résolution.

**11.** Le détecteur de la revendication 1, dans lequel un rayon de la fibre à basse résolution est supérieur à un rayon de la fibre à haute résolution, et/ou un espacement entre les fibres à basse résolution est supérieur à un espacement entre les fibres à haute résolution.

**12.** Le détecteur de la revendication 11 dans lequel la couche à haute résolution a une largeur égale à une taille maximale d'un faisceau de rayons X à spot de balayage incident sur l'écran de scintillateur (2206).

**100**

103

101

105

105

102

FIG. 1A

(PRIOR ART)

**FIG. 1B**
**(PRIOR ART)**

FIG. 2
(PRIOR ART)

FIG. 3A
(PRIOR ART)

FIG. 3B

FIG. 4

**FIG. 5**

**FIG. 6A**

EP 3 908 670 B1

FIG. 6B

**FIG. 6C**

EP 3 908 670 B1

400

403

701

403

702

## FIG. 7A

Low Energy WSF
712

Scintillator 716

X-ray Filter 710

Scintillator 716

High Energy WSF
714

## FIG. 7B

FIG. 8

EP 3 908 670 B1

FIG. 9

FIG. 10

FIG. 11

**FIG. 12**

**FIG. 13A**

FIG. 13B

FIG. 13C

**FIG. 14A**

**FIG. 14B**

**FIG. 14C**

EP 3 908 670 B1

FIG. 15

FIG. 16

**FIG. 17**

**FIG. 18A**

**FIG. 18B**

EP 3 908 670 B1

FIG. 18D

FIG. 18C

**FIG. 19A**

**FIG. 19B**

**FIG. 20A**

**FIG. 20B**

FIG. 21A

**FIG. 21B**

EP 3 908 670 B1

FIG. 22A

FIG. 22B

Low resolution
region number

1  2  3  4  5  ...

High resolution
fiber

12345678

2210

2206

Scintillation
screen

FIG. 22C

FIG. 23

EP 3 908 670 B1

## Multi-channel PMT

| | | | |
|---|---|---|---|
| 1 | 5 | 1 | 5 |
| 2 | 6 | 2 | 6 |
| 3 | 7 | 3 | 7 |
| 4 | 8 | 4 | 8 |

High
resolution
channels

Low
resolution
channels

**FIG. 24A**

EP 3 908 670 B1

2400

FIG. 24B

**2500** ⬎

| |
|---|
| detector layer |
| scintillator layer |
| high-resolution WSF layer |
| scintillator layer |
| low-resolution WSF layer |

2502
2504
2506
2508
2509

**FIG. 25**

EP 3 908 670 B1

FIG. 26A

FIG. 26B

FIG. 26C

1/8" (3.2 mm)

1/4" (6.4 mm)

1/2" (12.7 mm)

3/4" (19.1 mm)

**FIG. 26D**

FIG. 27A

FIG. 27B

2802

Positioning a first plurality of wavelength shifting fibers to define the at least one high resolution layer

2804

Establishing a variability of the first predefined signal response by changing a first space between each of the first plurality of wavelength shifting fibers

2806

Binding together the first plurality of wavelength shifting fibers using molded sheets of a transparent, flexible plastic binder

2808

Embedding scintillator powder between each of the first plurality of wavelength shifting fibers to form the at least one high resolution layer

2810

Positioning a second plurality of wavelength shifting fibers to define the at least one low resolution layer

2812

Establishing a variability of the second predefined signal response by changing a second space between each of the second plurality of wavelength shifting fibers

2814

Binding together the second plurality of wavelength shifting fibers using molded sheets of a transparent, flexible plastic binder

2816

Embedding scintillator powder between each of the second plurality of wavelength shifting fibers to form the at least one low resolution layer, wherein the first space is less than the second space

**FIG. 28**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6078052 A, DiFilippo
- US 73269933 B, Katagiri
- US 5302817 A, Yokota
- US 7067079 B, Bross
- US 2016170044 A
- US 6407392 B
- US 9618630 A
- US 5289510 A
- US 9069084 B
- US 5764683 A, Swift
- US 7099434 B, Chalmers
- US 163854, Rothschild

### Non-patent literature cited in the description

- **MOISEEV et al.** High efficiency plastic scintillator detector with wavelength-shifting fiber readout for the GLAST Large Area Telescope. *Nucl. Instr Meth Phys. Res. A.*, 2007, vol. 583, 372-81
- **YOSHIMURA et al.** Plastic scintillator produced by the injection-molding technique. *Nucl. Instr Meth Phys. Res. A.*, 1998, vol. 406, 435-41